# EUROPEAN PATENT APPLICATION

(11) **EP 3 705 584 A1**
(43) Date of publication of application: **09.09.2020**
(21) Application number: 19160908.0
(22) Date of filing: 05.03.2019
(51) Int. Cl.: C12Q 1/6886

(54) **METHOD OF IDENTIFYING PATIENTS WITH BORTEZOMIB RESISTANT MULTIPLE MYELOMA AND OTHER BLOOD DISEASES**

(71) Applicant: Max-Planck-Gesellschaft zur Förderung der Wissenschaften e.V., 80539 München (DE)
(72) Inventor: MÜLLER, Roman-Ulrich, 50937 Köln (DE); ALLMEROTH, Kira, 50933 Köln (DE); HORN, Moritz, 52355 Düren (DE); DENZEL, Martin Sebastian, 50668 Köln (DE)
(74) Representative: Arth, Hans-Lothar

(57) **Abstract**

The present application relates to an *in vitro* method and a diagnostic kit for identifying resistance to bortezomib treatment in a patient in need thereof by detecting a substitution in the amino acid sequence of the polypeptide β5 (PSMB5). The present application also relates to a composition and preferably to a synergistic composition of bortezomib and a further drug suitable for the treatment of a patient suffering from a disease selected from the group comprising multiple myeloma, mantle cell lymphoma, non-Hodgkin's lymphoma TEMPI syndrome, light chain deposition disease (LCDD), IgG₄-related disease, and Scleromyxedema.

SEQUENCE LISTING

<110>  Max-Planck-Gesellschaft zur Foerderung der Wissenschaften
       e.V.

<120>  Method of identifying patients with bortezomib resistant multiple
        myeloma and other blood diseases

<130>  GAR-P04151EP

<160>  15

<170>  PatentIn version 3.5

<210>  1
<211>  792
<212>  DNA
<213>  Homo sapiens


<220>
<221>  gene
<222>  (1)..(792)

<220>
<221>  gene
<222>  (1)..(792)
<223>  human PSMB5 nucleic acid

<400>  1
taccgcgaac ggtcgcacaa cctctctggc gatggccact tggtcgcgcc caaaaagcct      60

gaacccccag cacgtctaga cgacctagat ccaggtccct cagagtcact accagactcg      120

gaccggcgcg gtccgacccc acagggtctt ctcggtcctt agctttacga agtaccttgt      180

tggtgggacc ggaagttcaa ggcggtacct cagtatcaac gtcgactgag gtcccgatgt      240

cgcccacgaa tgtaacggag ggtctgccac ttcttccact atctctagtt gggtatggac      300

gatccgtggt accgaccccc gcgtcgccta acgtcgaaga cccttgccga caaccgagcc      360

gttacagctt agatactcga agctttattc cttgcgtaga gacatcgtcg acggaggttt      420

gacgaacggt tgtaccacat agtcatgttt ccgtaccccg acaggtaccc gtggtactag      480

acaccgaccc tattctctcc gggaccggag atgatgcacc tgtcacttcc cttggcctaa      540

agtccccggt ggaagagaca tccaagaccg agacacatac gtatacccca gtacctagcc      600

ccgataagga tactggacct tcacctcgtc cggatactag accgggcagc tcggtagatg      660

gttcggtgga tgtctctacg gatgagtcct ccacgtcagt tggagatggt gcacgccctc      720

ctaccgacct aggctcagag gtcactgtta caccgactag atgtactctt catatcaccg      780

agatggggga ct                                                          792


<210>  2
<211>  18678
<212>  DNA
<213>  Homo sapiens

<220>
<221>  gene
<222>  (1)..(18678)
<223>  huPSMB5 gene NC_000014.9

<400>  2
atggccgacc tcacttccct tacgcaacat ggacgttttc agtcacttcc tgtgattttg      60

aggttattct gatccaggac tacttgcatg accaaagcca agatggcagg tacgtccttt     120

tagaatttaa ggttagtgaa gccatctatt tccccgaccc ccttcagtga aaatggtctt     180

tcgcatctgg ctcttctttt tggaggcgtg cttgccagca gtcaaaatgg ctccattccg     240

gaatagattt aataggaagt gaagctgtga cggcgaggcg ttgcccggcc tatctttgct     300

aggcgttctc agaattagtt ctttctgccc acactagaca tggcgcttgc cagcgtgttg     360

gagagaccgc taccggtgaa ccagcgcggg tttttcggac ttgggggtcg tgcagatctg     420

ctggatctag gtccagggag tctcagtgat ggtctgagcc tggccgcgcc aggctggggt     480

gtcccagaag agccaggaat cgaaatgctt catggaacaa ccaccctggc cttcaaggtg     540

tggagccagc ccccttgcca ggctgagtac tgaacgcccg cgacttgcct ggcctcccag     600

cctgaccgga gtttgcggtg gccttggcca ccaacccagg cggaggctgg gtcctctcgt     660

tcggaccgca gcagttttgc tgtctcattg gcccaggagg cccctgtctt tgctctggtt     720

ggaaagggtg gcggtggggc cgggggtaag tcgggggagg aaggtgaggc ctcttgggtt     780

gattcctagg cgaccccagg ttgctgctgt tcaggatctt ctagctggga aaggccgcaa     840

caacaatgaa tcttggccga gggaaggcta ttgtaaacgt ttaccaaagt tttggaaact     900

gggcggcccc tcaaggcagg ggtaactgaa actggcatga aggggaagcg tggtgagggt     960

aagcattgta ctaagaatca gacatttggg gttccttgcc cagctctgca ataactagct    1020

ctagattgag aaatcatttg atttcattga acctgtttcc tcatctataa aatgatttgt    1080

tttttttttt tttttgaaac agggcttttc gctgtcgctc aggctggagt gcagtggctc    1140

cagcgcctca gcctccagag tagctgacac tataggcaca caccaccacg acaggctaag    1200

tttttttact ttttgtagag atggatctcc ctgtgttgcc caggctggtc tccagcacct    1260

ggcctcaagc ggtcctcccg ccttggtctc ccaaaatgcc tgggttacag gagtgagcca    1320

ccacgcccat cctataaaat gaatttaata tgggttatat ccatgtcaag ggattgatgt    1380

gaagctcggg tgacattaat atatggtgaa gtatagaagt atactgtgaa gtataaaagg    1440

acggagaaag agatgtgctg ggtttggatt gatgcaaaaa gaagtatgta ggagtgtttt    1500

tgtggtctta tgtggcctgt tttgtgtttt cctctgatct taacagttcc gccatggagt    1560

catagttgca gctgactcca gggctacagc gggtgcttac attgcctccc agacggtgaa    1620

gaaggtgata gagatcaacc catacctgct aggcaccatg gctgggggcg cagcggattg    1680

cagcttctgg gaacggctgt tggctcggca atgtcgaatc tatgagcttc gaaataagga 1740

acgcatctct gtagcagctg cctccaaact gcttgccaac atggtgtatc agtacaaagg 1800

catggggctg tccatgggca ccatgatctg tggctgggat aagagaggcc ctggtgagtt 1860

aagctgcaac cacatgatcc ttgggctgac actacagtga ggaggggttg gatggacaga 1920

tgaattaaag ggcttggtgt caatgctgaa gaagtgtaat taggtcctta gcccttcctt 1980

ctctcttttt ttttttcctg tttttttaga ctgtgtctca ttctgtcgct caggctggag 2040

tgcagtggca tgatctcagc tcactgcagc ctccgccccc aggttcagga attcaagacc 2100

agcctggcca acatggcgaa accccatctc tactaaaaat acaaaaatta gcccggcgtg 2160

gtggcgggcg cctgtaatac cagctgctca ggaggctgag gcatgagaat tgcttgaacc 2220

caggaggtgg atgttgcagt gagcagagat cgcgccactg cactccagcc tgggcgacag 2280

agtgagactc tgtctcaaaa aaaagaattt aagaattcgt tgaaggccgg gcgtggtggc 2340

tcactcctgt aatcccagca ctttgggagg ctgagacagt ggatcacgag gtcaggagat 2400

cgagaccatc ctggctaaca aggtgaaacc ccgtctctac caaaaataca aaaaattagc 2460

caggcgtggt ggcaggcgcc tgtagtccca gctactcagg aggctgaggc aggagaatag 2520

tgtgaaccca ggaggcggag cttgcagtga gtcgagatgg tgccactgca ctccagcctg 2580

ggcaacagag cgagactctg tctcaaaaaa aaaaacaaaa aaaagaattc gttgaatccc 2640

acatgtggca gtagtgctat ttcaagtgtt cattgaccat gtgtggcttg tggctactgt 2700

attggactct aatatatggt tgtccctcaa aagttagtca tttctcttct gtttttttct 2760

tttttctttt ttttgagatg caaagtttca ctccgatcgt ccagtctggc gtgtaatggc 2820

attgtttcag ctcactacaa cctctgcctc ctgggttcaa gtgattctcc tgccgcagtc 2880

tcccaagtac ctgggattac aagcgcccac cagcatgccc agctaatttt atatttttag 2940

tagagatggg gtttcaccat gttggccagg ctggtctcga aatcctgacc tcaggtgatt 3000

cacccgcctc agcctcccaa agtgccagga ttaaaggcgt gagccgctgc acccggcctc 3060

ttctcttttt ttctttagag agtctttggc ctagaagggg ccttttgggc tcctctcttt 3120

tttgaggggg tggggatgga gtctcgctct gtcgccccga ctggagtgca gcggcgggat 3180

ctcagctcac tgcaatctct gcctcccagg ttcaagtgat tctcctgcct caaccttcca 3240

tgtagctgag actacaggca tatgccacca tgcgcgtcta atttttgtat ttttagtaga 3300

gacagggttt caccatgttg gccaggctgg tcttgaactc ctgacctcag gtgatctgcc 3360

cacctcagcc tgccaaagtg ttgagattac aggagtgagc cattgcgcct agtcctgggc 3420

tcatttcttt tatttctttg ctggttataa tcacccttac aatcatgcta ggtccggttc 3480

tatcatctct gaaaataagt tccttagcct gtttttcctt tccagcattt aagaaacttg 3540

ccagacagtt caactttgta tttaatgtca tttcctcatc agccagtttt gaattgcccg 3600
tttagtaaag ttggagtata gctagtcacc ttctaatttt cccttccata tgcttaaaaa 3660
taattattct ttcctccttt tgattttgct tcattcattt acctgcttac taaataccta 3720
gttatcttat agaacaatta tagggtacct accacttttt cacttgcttt caaacgagaa 3780
tagaaagggc tgtagggagt atccacttac tacccaatgg aggatgaaag gaaattaaaa 3840
accggatgtg agagaggagc tcactttgtt actttggaaa ttgagatgct tttctctgaa 3900
aggtgggaaa agacagttcc atacatttta cgtgatccag ggaagcattt tggaaatgtt 3960
gctttgaacc tttagctggc ggatttgttc taggtgatgg aaacagtgta aaagagtaca 4020
gggaccgggt aacaaggaaa atccagggag actgagcatc atttaaacag tgccttcttc 4080
aagaggcctt cctgatgacc ctgtcccatt cattgtgagt cagccccttg cctcatatta 4140
cagttggccc ttgagcaaca tgggtttgaa tcactcaggt ccacttatac gcaggtattt 4200
ttcaacctaa ttctgattga aaatacacat tcccagaatt tgaaacccaa atatacagag 4260
gaccaattta tttatttatt tatttattta tttttgagat ggagttttcc tcttgtcacc 4320
catgctggag tgcaatggcg cgatctcagt tcattgcaac ctccacctcc tgggttcaag 4380
tgatctcctg cctcagcctc ccgagtagct gggattacag acacccgcca ccctgccagc 4440
taacttttgt atttttagta gagacagggt ttcaccatgt tggccaggct ggtcttaaac 4500
tcctgacctc gggtgatcca cctgcctcag cctcccaaag tgctgggtgg cgtgagccac 4560
catatctggc caacttttca tatatgcagg ttctgcaggg ccaactttgg gacttgagtg 4620
tgcatagatt ttggtatatg caggggtctt gggacaaatc cccttcatat accaaaggat 4680
gactagttaa cccaaggctg aataagataa ataacctttt ttttttttta ttgagacgga 4740
gtctcgctct gtcgcccagg ctggagtgca gtggtgccat ctctgctcac tgcaagctcc 4800
gcctcccgga ttcacgccat tctcctgcct cagcctcccg agtagctggg actacaggtg 4860
cccgccacca cgcccggcta attttttgta tttttagtag agacggggtt tcactgtgtt 4920
agccaggatg gtctcaatct cctgacctca tgatccaccc gcctcggcct cccaaagtgc 4980
tgggattgca ggcgtgagcc acagctcctg gcctgataaa taccgttttt aaaagaacca 5040
gttggctggg catggtggct catgcctgta atctcagcac tttgggaggc cgagatgggt 5100
ggatcaccgg agatcaggag ttcgagacca gcctgaccaa catggtggaa ccttgtctct 5160
actaaaaata caaaattatt gggaggccga ggcgggcgga tcacgaggcc aggagatcga 5220
gaccatcctg gctaacacgg tgaaacccca tctctactaa aaatacaaaa aattagctgg 5280
gcgtggtggt ggtcgcctgt agtcccagct acttgggagg ctgaggcagg agaatggcgt 5340
gaacccagga ggtggagctt gcagtgagct gagatcgtgc cactgcactc cagactgggc 5400
aacagagcaa gactccatct caaaaaaaaa aaatacaaaa ttagccggct gtggtggcac 5460

atgcctgtaa tcccagctac tcagggagat gaggcaggag aatctcttga accctggagg 5520

tgcatgcctg taatcccagc tactcaggag gctgaggcag gagaatcaat cacttgaacc 5580

tgggaggcag aggttgcagt aagctgagat tgtgccattg cactccagct tgggaaacaa 5640

gaaagaaact ttgtctcaaa aatcagtcag tcaatcaata aataaaaaga accagtcaaa 5700

tgcggaagga gttttgagga ggaagagatt acttatgatg ggaggaattg gggaagactt 5760

catggattgg tggtggttgt cctggatgta aaaaagggaa agggaacata acaaagaaag 5820

ggaggaaaga agcatgagct tttgaggagt aactgaaagt ccagtttaat tggaactaag 5880

tgggtgaagg ggaatagtga gccataaagc cagaagggta ctttggaacc agatgatgac 5940

ccccctctag gcaagagagc atggcactgg ggctgaggag gggccagaga aaggagtaag 6000

gtagacatca gaatcacctg aacctgaaaa ccaataaggg cagaggtgct caagctggct 6060

tgcattaaaa tcacctgggg gacttaaaaa acaagttcca tacctgtttc ccaccccaca 6120

ccagttataa aaaataccta gggtaagtcc tgtgcatttt tgaaaaaaat ctttgttaga 6180

tgactctagt gcacagtgag atgtgagaat cactgaaaca ggaaaaagag tagtgatgct 6240

attggcagaa tttcagaaga caggaggaag cgtgggtttg tggcgcacaa gagagatgag 6300

tgtggtaggt aatggagaac cactggtatc taagtttagg aagtttgaat ctgtacatga 6360

tgacaaagac atccagctgg agggatccag taggcagttg ggattgaaca ctttgtctaa 6420

attttcatct gtaccaaaag tcagctagtt ttttcataaa gggccaaata gtaaatattt 6480

taggtctgca ggcctaaatc tttgtctcaa atacttaact ctgccggtgt tgcacaaaac 6540

aaccatagac agtatggaaa cagtggctct gttccaataa cattttaatt acaaaaataa 6600

aggcagttgg cctggccaaa ccctggtcta taccagcaat agaaatataa tgtgagccat 6660

atatgtaatt ttacatttta tactagctac gcttttaaaa ttaaaaaaaa ttaatttcaa 6720

tatattttgt ttaacctaat aaatccaaaa tattatgtta gcaggtagta ctagttcatt 6780

tcaaatgctc aactagtcct gtgtggctag tggctaccat atggacagtg caggtcttta 6840

caatgtgatt acaaactcta ggctttatta tcctcctgtg taaagtatgt cttgttctcc 6900

agatagtcta aactctttga gttctggcat tatggagttt gtttttctgt cacacaggct 6960

gcagtgcagt ggcataatca cagctcactg tagcctcaac ctcctgggct caagtgactg 7020

ggattatggg gtcttttgat gggggagtgg ggattatgtt ttgtttgttt gttttttgag 7080

acagagtttc actcttgttg cccaggctgg agtgcagtgg tgcgatctcg gctcactgca 7140

aactccacct cccaggttca agtgattctc ctgcctccgc ctcctgagta gctgggatta 7200

caggcatgcg ccaccacgcc tggctaattt tgtattttta gtagagacag ggtttctcca 7260

tgttggtcag gcttgtcttg aactcctgac ctcaggtgac ccacctgcct cggcctccca 7320

aagtgctggg attacgggtt tgagccactg cgcctggccg gggattatgt tttaaatgtt 7380

atctttcaca gtgtctgaag ttctgtgctt gaaacctaag tcatttggaa tgtacttgtt 7440

ttgtgggtgt gctgagagga tcggcaacat ggcaaggtag ttattataat ataaggtgag 7500

atggggtggt atgttgtaga atcctggagt cttaatatta aattttattc cttcaggttt 7560

tttttttttc ctgaagacag catctcagtc tgttgcccaa gctggagtgt ggtggtgtga 7620

ttatagctta ctgcagcctg caagtcctga gctcaaacaa tcctcctgtt tcaacctccc 7680

acagacatgc accaccacac tcagctagct tttaaaaaaa taataataat tttatagaag 7740

tcctatcttg tatttatgtt ggcttccctg ggtttctctt ttatatatat atatattatt 7800

ttatttattt attattatta ttattatttt tttttttttt gagacaaagt cttgctctgt 7860

ctcccaggct ggagtgcagt ggcgcgatct cggctcaccg caagctccgc ctcccgggtt 7920

tacgccattc tcctgcctca gcctcctgag tagctgggac tgcaggcgac cgcaaccaca 7980

cctggctaat tttttgtttt ttagtagaga cggggtttca ccgtgttagc caggatgatc 8040

tcgatctcct gacctcgtga tccacccgcc tcggcctccc aaagtgctgg gattacaggc 8100

gttagccacc gcgcctggcc tatttattat attattttga gacagcgttt cactcttgtt 8160

gcccaggctg gagtgcaatg gcgcgatctt ggcttaccgc aacctttgcc tcccaggttc 8220

aagtgattct cctgcatcag cctcctgagt agctgggatt ataggcatgt gccaccacgc 8280

ctggcaaatt ttgtattttt agtagagatg gggtttcttc atgttggtca ggctggtctc 8340

aaacgcctga cctcaggtga tctgcccacc tcagcctccc aaagtgctgg gattacaggc 8400

atgagccacc gcgcccggcc tattttttta tttttttaaa ttttattcta ggttttaaga 8460

gaactactgc tggccgggcg aggtggctca cgcctgtaat cccagcactt tgggagtccg 8520

agaagggcag atccccagag gtcaggagtt caagaccagc ctgaccaaca tggtgaaacc 8580

ccgtctctac taaaaataca aaaattagct gggcgtggtt acgagcgctt gtaatcctag 8640

ctacctggga aactgaggca ggagaatcac tttaacccag gaggcggagg ttgtggtgag 8700

ccgagattgc gccattgcac tctagcttgg gcaacaagag caaaactcca tctcaaaaaa 8760

aaagaactac tgccttggca tatgaattag atatcaaaag gggtgatctt tttttttttc 8820

ctgctaacct catctccctt tccaggcctc tactacgtgg acagtgaagg gaaccggatt 8880

tcaggggcca ccttctctgt aggttctggc tctgtgtatg catatggggt catggatcgg 8940

ggctattcct atgacctgga agtggagcag gcctatgatc tggcccgtcg agccatctac 9000

caagccacct acagagatgc ctactcagga ggtgcagtca acctctacca cgtgcgggag 9060

gatggctgga tccgagtctc cagtgacaat gtggctgatc tacatgagaa gtatagtggc 9120

tctaccccct gaaagagggt ggatgcagct gcttgtgttt cttggggtga ctgtcattgg 9180

taatacggac acagtgaccc atcctccatc ctatttatag tggaagggcc ttcaattgta 9240

tcagtacttt tttttaagct ctggcacatt gacctctatg tgttaccagt cattaatgag 9300

ctgctgcaga ggtgactatt tgttttactt tcttggatgt taacattaca ctactcacta 9360

ctcaatctca gcctgtgttg catcttttgt ccacactgtc cctcctccaa gcacttttca 9420

gtcaaataag ccaggacagt ggaagggaag tgactgtaat tacaggaaac agtggtgtga 9480

agacattgtc tagtgaatac attaatgtcc ccactcatga agctaataac ggagaaggag 9540

agagaggcag ggaggaggga gacacttctc gatttggttt acaaagagat gaaaagttga 9600

tgaggtggaa agatgcacag ggctgtacca ggcagggctc cagcggaaaa ggctgtcacc 9660

ctgtggtggg ggaagggagg ctgaggctac atgcagagcg gaagggtgaa gagggtaaat 9720

cttgactctt aaaccaactg ggaagggaag gcttggaccc tgggaagggg aggacaaaga 9780

agtggctgtg aaagcagtag taaccacaag cttggtgacc cttgtccttt tggtcctcca 9840

gtgcatatgt gtggagggga gagggtagtg atggtgattt tatagcctcc tggtcaggaa 9900

gcagcactaa attgtcactg gaatgtgtgg gtggggaaga ggccctgctg agagcagaga 9960

gatggctgat ttatcacacc cagcactgtt ggggctgggg ggagttccca agctatggtg 10020

gcagcagatg gcaaggcctt ctgttaaaga gaagacaggg gatagaggag gttgatggga 10080

gaaaccttta aaaaatggga gcggaggaca caaacaatac caccttgtat ttagtcagaa 10140

ggcagaaagt ggttggtgaa ggtgacaacc cagtgtcatg ttttgagact gagaggtttg 10200

gagaagtcat tcacagaaga agagggggta aaccctcctc tcctgtactg cagctacctt 10260

cccttccccc tcagccttgt gcagtcttcg tattgattga tgagggctgt cggccaggaa 10320

ctgatcgagg cttgttaatt gcatttgtca aatgcaggga aattgggaat tagtgaaatc 10380

ggagaagggg gtttggaaaa caaatgactc gtgcctaagg aaattttttg caggaaagta 10440

tctcaggagc ccctgcagtc agggagctgc tggtgtggac tcagactaca tggttgaaat 10500

aggcaggagc tgggcggggc acagtggctc aggcttgtaa tcccagcacc agcactttgg 10560

gagacggagg caggcagatc acttgatgcc aggagtttga gaccagtctg gccaacatgg 10620

tgaaacctgt ctctactaaa aatacgaaaa attagctggg tgtggtggca ggcacctgta 10680

atcccagcta cttgggaggc tgaggcagag gttgcagtga gccgagatgg tgccattgca 10740

ctccagcctg ggcaacaaaa gcaaaactcc atctaaaaaa aaaaagaaag aaagaaatag 10800

gcaggaccca agctacatct tccttctcgt agactgttgg gaaatggtta gctgcgtgag 10860

aagcacaggt aggtcatctt cctcccactt aggacattgg tttgcagatc ttaaaaaaaa 10920

aaaagaaaaa aaaaaaagca ctcttaggag aaatcccaat gcctgaaaaa aaaatcagag 10980

gttgaaattg agctgagagg tccagagcct ccactattga gcttccccat ccagccactc 11040

cccgcagcac cctttgttag ccccatacac atacacagtc cctggcactt cttggaacct 11100

caaggaacac aatttgaaca cctcttccct gggaaatagc cttaaggata gaaagagaat 11160
tgaagattgg aatttcctta catgacccca gagagcataa gaccccagct ttggtccatg 11220
aacagaaaat tcattctgga gagaagagat gatcgtgaaa caatgcaacc ctttgccctg 11280
tttctgtctc acactttgct ttggcagtgg tttagctgtt taacggaaac acctattctg 11340
cactggcaga gtccttatgt tagaagagaa aatgcatgtt ctgttctggc atatccctgt 11400
tgatctacct tcctgggtct tgggtaaaag actcatttaa tggaagagct cgatttgtgg 11460
taggtttttc tgggaagaaa gtagagcaga ggtagggaaa aactgcaagt ctgtaggcta 11520
gtggtatcca ccctgtgcat ttgtgatttc ttgctgatgg ttactggggg gaaattggct 11580
ttgcagcgat gtgtgtctcc gcttgtgtgg gaatgatgga tgccgcatag gattggagag 11640
atgtttttga gtggagtgta ccagttcagg gactcccatc agattcattt cccagtacag 11700
tcctcttgac aaaggatgat ggaaaagaaa tcgccttaaa tagctggaag attaatttct 11760
ctcagcagtt cccaggcgtc caagggactg ggctctttaa gtgatgtctg acaacttctc 11820
ttccctcaac agataactca gtaacaccaa aatctggctc ctcggggtag gtgatgggac 11880
taatctgatt aaatagggag caaatactcc tggaggcccc ttgctttctc ttgctagcct 11940
tccagcatct gcacaccagg caagggaggg ctgtggggag tagcagtgca ctgtggggtg 12000
agtagaatgt tcatgaagga aggtgaaaag gtaggtgagg atgtggggga ggagggagga 12060
agggcacagt taacgcaaat caaagactga aggataatag ttactgagtg ttcctcttta 12120
ggcaaaacca gaaggtcagt gtctcttgat atcctcctca gctagcaaat gagaaaactg 12180
tcagagccat ttggggagtg gaaggtcaca tcctccgagg ggaaataaaa taactgattt 12240
attctggccc acggattcta tgtggtctgt taggggaagg aagaaagagg tgggatgaaa 12300
agagaataaa gtagaaaaaa aaaggtggat ggaaattttg aggggagcat ttcccttgtg 12360
tcaggagtaa caaggatagg atgattgggt taggagagaa tctacatgac catatctaat 12420
tttcggtgga tacatttagg tttttaattt tcttttattt ttgtttcttt tgttagatct 12480
tcccctgacc tgctcccagg gctataacag atacggactt tgaaactgta tggagtatag 12540
agcagtgctc tataatttaa aatatacata tattgcaacc aagcacctct gctgttttgc 12600
gtttgtttct tgtttgtttt tataaattct agtttccctt caggaagcca gaatcatact 12660
gggaatgctt taattatagt gggcaagagg gagctcaaac agcaatcaga actgccctga 12720
tcagcagctg ctgccacctt ctacctgacc tgggccaccc ctctttttcc ccccaagtca 12780
gcctcccgtg ctccccaggc gtggcttcag agccaagttc tggtttccag gagctctgcc 12840
cccactgaag catttcaata aaaaacattt ctaaaatgtc ccctttgcag ttgttagtgg 12900
gtcaagcaga agaaattcta cccacgtcag cttcttgggc ttatttgacc caactcattt 12960
ctcccagaag cttttgcctc tgagttaggt gccaagctta agctggtatt taactcttta 13020

tggtccagat taagctgtca tcagccataa agatatgacc cagtttattt gaaaagaaat 13080

aagacaccat gggttcgaca cgttctgatg cttcccatta cttatccccg gccccatctt 13140

aggctgttcc tccctttgcc tcgttccatg ttttctctct gctctctcac tttataaaaa 13200

ttctctttcc tttgcagagt ttaaataaaa accaatcaat caataaacca agggctgctt 13260

ataggcttta ctgtaacaag aaaaggttat cattaactgc agggtaccca aaaagctggt 13320

attatagagg aaaacagatg gaatggcaga aagtgaacta gagagaacaa gagacaaacc 13380

agagacaaac aaaagcgact cccagacagc aagcgcgaga ttgagcaaag tgcagaggga 13440

gatgtgaacg tggcagagaa agatggatgg agatattgac aggcagagct ggactgtgaa 13500

tgggggcaga cagggagtgg gaaaggagac taagaaggac agtgatagag agagtgggca 13560

gaaaaacagg gaggaaggaa gcggctcgaa gctaatgcac agtgaaacat gtccccctgg 13620

agacagataa tttctccagc catacgtaat gaacacgtgc ctctctgtgt gttgcccctg 13680

tactgtcaca gtcacttaaa ttctgcaaga atctctccat gtctacacca cttatgcgag 13740

gttggaaaga aggtttgggc tctcacaacc cccatctcaa tccagccagg ccgaagacag 13800

aaaattagaa acctgtgtcg taaaagtaac agggtgagtg aggcaggatc acagatccag 13860

taccttcgaa aatttgtttt taggttcact aagaagcctt ctctccccac acctccatac 13920

tccctggcaa cacacatcat acacacataa ctcatcctct tttctgcaaa catgcctgca 13980

gatcagggcc tagggtgcct aagtcaggag agggaagcca ctacctccca ggaagactga 14040

ggaatagcac atgtcaggta agacgagaaa taagtaggtc caaatagaag taggtgtagc 14100

ctacaaggca ccaaggtaga ggggatgttt ttgcacgacc gcagctcttt tctcattcta 14160

gcctcaacac catcactatc ccagagacac ctatgcactc taagcctccc catgcataca 14220

gagtttcaca caatacacag gctttttttc acagcagctt atctccagcg gtgatgtgtg 14280

gtattgtctg attagaggga atcctctgag caccatggtt atgtgggagg gctcataatc 14340

accatgtctc ccttgagtct tgagtttgcg ctcatagctt agtctttaga aaagaaagca 14400

atggagcatt ttaaaccaac acaacatatt cagcatagtc ctgcatttcc aagcacctcc 14460

taaagaggaa aaatgcataa aggtggatca atccaagtgc ggtccttggc taggggtcag 14520

cactgttcta catctcattt aaacagcctt agggccaggt gccatggttc acacctataa 14580

tcccaacact ttgggaggcc aaggtgggag gatcacttga gctcaggagt tcaagaccat 14640

ctgggcaaaa taatgagacc cccatctcta caaaagatat taaaatttag ccaggtgtgg 14700

tggtacatac ctctagtacc agctcctcag gaggctgaag caaaaggatc acttgaacct 14760

gggacatgag gctgcaggga gctgtgatcg caccactgca ctccagcctg ggtgacagag 14820

ttagaccctg tctcaaacaa aacaaaacaa atcaaaaaac agccttaggc atgtgccaca 14880

tcaaacagca tctatgtagt gtctctggtt gtttcctggc atgtctgcct gctttttcag 14940

ttaagcacta tagtgtagtt tttacttgtt cccccacagt gtctagcatg ctgttggcta 15000

ctcaggaaag atatataata aatgtacttt taataaatga attaatgagg ctaggtgcat 15060

tagctgacgc ctgtaatcct aacactttgg gaggccgagg tgggtggatc acttgaggtc 15120

aggagttcga ggccagcctg gccaacgtgg tgaaactctg tctctactaa aatacaaaaa 15180

ttagctgggt gtggtggtgt gcacccgtaa tcccagctac ttgggaggct gaggcaagag 15240

aatcacttga actgcagcgc aggctgcact gagcagagat tccactagcc actatactcc 15300

agcctgggca acagagcaag actccactac aaaaaaaaaa aaaatacaga attaatgaat 15360

gattgccagg cgcggtggct cacgcctgta atcccagcac tttgggaggc cgaggcaggc 15420

ggatcacctg aggtcaggag atcgagacca gcctggctaa cacagtgaaa ccccatctct 15480

actaaaaata caaaaaaaaa ttagccgggc atggtggcag gcgcctgtag tcccagctac 15540

tcgggaggct gaggcaggag aatggcgaga acccgggagg cggaggttgc agtaagcaga 15600

gatagcgcca ctgcagtctg gcctggacga cagcgagact ccgtctcaaa aaaaaaaaaa 15660

aaaaaaaaag accagcctgg ccaacacggt gaaacctcgt ctctactaaa aatacaaaaa 15720

ttagccaggc gtggtggcag gcacctgtag tcccagctac tcgggagcct gaggcaggag 15780

aatcgcttga acctgggatg tggaggttgc agtgagccga gattgcgcca ttgcactcca 15840

gcctggggaa caagagcgag acttcatctc aaaaaaaaaa aaaaaaaaaa aaattaatga 15900

atgatttcac tatggtgtca ttttgccagt ggttcctacc cttcttagca ctgtgactcc 15960

catattgttt aattttaaga tgccctttgc tgctcttaaa catactatgg gccaggcacg 16020

gtggctcatg gctataatcc tagtgctttg ggaggccaag gcaggtggat cacctaaatt 16080

caggagttaa aaccagcctg gccaatatgg tgatgaaact ctgtctctat taaaaataca 16140

aaaatcagcc aggtgtggtg gcatgcgcct gtaatcccag ctactcagag gctgaggcag 16200

gagaatcact taaacctagg gggcagaggt tgcagtgaac caagattgta ccacttcgtt 16260

ccagcctggg tgaaagagtg aaactctgtc tcaaaaaaac aaaaaaacaa aaacaaaaac 16320

aaaaaaacta tgaaacgtat attaattttt aaaattaaga caaattaaaa ttacttgttt 16380

tgtgaatttc tttttttttt ttttgagaca gagtctcgct ctgtcgccca ggcaacattg 16440

gcgcaatctc ggctcactgc aatctctgcc tcccaggttc acgccattct cctgcctcag 16500

ccttcccagt agctggggct acaggcgccc gccaccatgc ccgctaattt ttgttttttt 16560

tgtatttttt agtagagacg gggtttcacc gtgttagcca ggatggtctt ttttgttttt 16620

ttgggacttt ttttttttct ttttgacacc actgcactct agcctgggcg acagagcgac 16680

actccatctc aaaaaaaaag aaataacata aatgtaaaga agataatgaa gaaggcaaag 16740

aggagaaaaa tgagtagata gatgccagct attatatgta gaagaaataa cagaatttga 16800

aaaatcacca tttggcaaac cccaatgcaa aaataggaag gtgcaaggtt gtggaagaac 16860

aggatattca cattatctaa gataatcact gtattagttt gctaaggctg ctataacaaa 16920

acaccatagt ctgagtggct taaacaacag aaattacttt ctcccagttc tggaggctgt 16980

aggtccaaga tcaaagtatc agcagtgttg atttcttctg aagcctcagt ccccttggct 17040

tgcagagagc tgccttctca ctgtatcctc acatggtctt tcctctgtgc accacaggta 17100

tctctttgtg tccaaattcc ctcttcttat aaggacacca gtcagactgg ctctacccta 17160

agtgcctcat ttaacttaat cacttcttta aagactcagt ctccaaatac agtcgaatcc 17220

tgaggtactg gaggttaggg cttcagtata taagttttag gggaacacat ttcagcccat 17280

aacaatcact ctatttatta ttagtttact aattaattac aaatggcaaa tatacttcta 17340

caacagagac gtctagcaga catgaataaa cttgcagtca ccaataatag aacaaactga 17400

cattatatgc ctcttgatgt gatacaataa gaagtatata aaattaccta agtaatattc 17460

tcgctaaaaa tgtttaacct tggccctgat gtggtggctc atgcctataa ttccagcatt 17520

tggggaggcc aaggcaggag gatcccttga gcccaggagt ttgagaccag ccggggcaac 17580

atagggggat cttgtctcta ccaagtaata actttttaaa attagcaggg catggtggca 17640

tgcacttgta gtcccaagct actcaggagg ctgaggcagg aggatggcat gagctttgga 17700

tgtcaaggct gcagtgaact gtgattacat cactgcactc cagcctgggt gacagagcaa 17760

gaccctgtat caaaaaagaa aaaaaaaaaa aagcttaacc tgaacctaat cacaagaaaa 17820

cagataaatc agattgcaaa taatgtcctg actcttccag tagaaacacc acgaaaaatt 17880

cacacacacc cacatggcaa aggagcacag cacttaggtg cagtgctgca gaatcctgtc 17940

ccacgcattg caaatgagct tgttctcggt cttgggacac ttgtggatgt accctgtatc 18000

ttcacagtac ttgattttca cagtaactgg ttgccaagga gaaaaaccat aaaggcacct 18060

ataatcccag cactttggga ggccgaggtg ggtggatcat ctgaggccag gagttcgaga 18120

ccagcctggc caacatggtg agaccccgtc tctactaaaa atgcaaaaat tagccgggac 18180

tggtggtgca tgcctgtaat cccagctact caggaggctg aggcaggaga atcgcttgaa 18240

cccaggagac agaggttgca gtaagccgag atagtgccac tgcactccag cctgggtgac 18300

agagcaagat tcagtctaaa aaaaaaaaaa aattagttgg gcgtggtgac gggtgcctgt 18360

aatcccagct actcaggagg ctgaggcagg agaattgctt gaacctggga gggagaggtt 18420

gcagagccaa gaccatgcca ctgcactcca gggtgggcaa gagactctat ctcacaacaa 18480

acaaacaaat agaaaacata aggggtacaa tttcacaaaa tcattaggtt ttaagatgag 18540

tgtgtgtatt tgaaagtttt gtgatgattg tttcttttct tgtaggttga aagaagctca 18600

aaggcaagaa tgttactttt gaattcccaa agtttcaatt gtaaacaaaa atgactaaat 18660

aaaatatcat tcacagtt                                                    18678

<210>  3
<211>  21
<212>  DNA
<213>  Homo sapiens

<220>
<221>  primer_bind
<222>  (1)..(21)
<223>  huPSMB5exon2_fwd

<400>  3
acggagaaag agatgtgctg g                                                21

<210>  4
<211>  20
<212>  DNA
<213>  Homo sapiens

<220>
<221>  primer_bind
<222>  (1)..(20)
<223>  huPSMB5exon2_rev

<400>  4
agcattgaca ccaagccctt                                                  20

<210>  5
<211>  21
<212>  DNA
<213>  Homo sapiens

<220>
<221>  primer_bind
<222>  (1)..(21)
<223>  huPSMB5exon3_fwd

<400>  5
tcctgctaac ctcatctccc t                                                21

<210>  6
<211>  20
<212>  DNA
<213>  Homo sapiens

<220>
<221>  primer_bind
<222>  (1)..(20)
<223>  huPSMB5exon3_rev

<400>  6
cttcccttcc actgtcctgg                                                  20

<210>  7
<211>  204
<212>  PRT
<213>  Homo sapiens

<220>
<221>  PEPTIDE
<222>  (1)..(204)
<223>  human mature beta-5

<400>  7

Thr Thr Thr Leu Ala Phe Lys Phe Arg His Gly Val Ile Val Ala Ala
1               5                   10                  15

Asp Ser Arg Ala Thr Ala Gly Ala Tyr Ile Ala Ser Gln Thr Val Lys
            20                  25                  30

Lys Val Ile Glu Ile Asn Pro Tyr Leu Leu Gly Thr Met Ala Gly Gly
        35                  40                  45

Ala Ala Asp Cys Ser Phe Trp Glu Arg Leu Leu Ala Arg Gln Cys Arg
    50                  55                  60

Ile Tyr Glu Leu Arg Asn Lys Glu Arg Ile Ser Val Ala Ala Ala Ser
65                  70                  75                  80

Lys Leu Leu Ala Asn Met Val Tyr Gln Tyr Lys Gly Met Gly Leu Ser
                85                  90                  95

Met Gly Thr Met Ile Cys Gly Trp Asp Lys Arg Gly Pro Gly Leu Tyr
            100                 105                 110

Tyr Val Asp Ser Glu Gly Asn Arg Ile Ser Gly Ala Thr Phe Ser Val
        115                 120                 125

Gly Ser Gly Ser Val Tyr Ala Tyr Gly Val Met Asp Arg Gly Tyr Ser
    130                 135                 140

Tyr Asp Leu Glu Val Glu Gln Ala Tyr Asp Leu Ala Arg Arg Ala Ile
145                 150                 155                 160

Tyr Gln Ala Thr Tyr Arg Asp Ala Tyr Ser Gly Gly Ala Val Asn Leu
                165                 170                 175

Tyr His Val Arg Glu Asp Gly Trp Ile Arg Val Ser Ser Asp Asn Val
            180                 185                 190

Ala Asp Leu His Glu Lys Tyr Ser Gly Ser Thr Pro
        195                 200

<210>  8
<211>  263
<212>  PRT
<213>  Homo sapiens

<220>
<221>  PROPEP
<222>  (1)..(263)
<223>  human beta-5 propolypeptide

<400>  8

Met Ala Leu Ala Ser Val Leu Glu Arg Pro Leu Pro Val Asn Gln Arg
1               5                   10                  15

Gly Phe Phe Gly Leu Gly Gly Arg Ala Asp Leu Leu Asp Leu Gly Pro
            20                  25                  30

Gly Ser Leu Ser Asp Gly Leu Ser Leu Ala Ala Pro Gly Trp Gly Val
        35                  40                  45

Pro Glu Glu Pro Gly Ile Glu Met Leu His Gly Thr Thr Thr Leu Ala
    50                  55                  60

Phe Lys Phe Arg His Gly Val Ile Val Ala Ala Asp Ser Arg Ala Thr
65                  70                  75                  80

Ala Gly Ala Tyr Ile Ala Ser Gln Thr Val Lys Lys Val Ile Glu Ile
                85                  90                  95

Asn Pro Tyr Leu Leu Gly Thr Met Ala Gly Gly Ala Ala Asp Cys Ser
            100                 105                 110

Phe Trp Glu Arg Leu Leu Ala Arg Gln Cys Arg Ile Tyr Glu Leu Arg
        115                 120                 125

Asn Lys Glu Arg Ile Ser Val Ala Ala Ala Ser Lys Leu Leu Ala Asn
    130                 135                 140

Met Val Tyr Gln Tyr Lys Gly Met Gly Leu Ser Met Gly Thr Met Ile
145                 150                 155                 160

Cys Gly Trp Asp Lys Arg Gly Pro Gly Leu Tyr Tyr Val Asp Ser Glu
                165                 170                 175

Gly Asn Arg Ile Ser Gly Ala Thr Phe Ser Val Gly Ser Gly Ser Val
            180                 185                 190

Tyr Ala Tyr Gly Val Met Asp Arg Gly Tyr Ser Tyr Asp Leu Glu Val
        195                 200                 205

Glu Gln Ala Tyr Asp Leu Ala Arg Arg Ala Ile Tyr Gln Ala Thr Tyr
    210                 215                 220

Arg Asp Ala Tyr Ser Gly Gly Ala Val Asn Leu Tyr His Val Arg Glu
225                 230                 235                 240

Asp Gly Trp Ile Arg Val Ser Ser Asp Asn Val Ala Asp Leu His Glu
                245                 250                 255

Lys Tyr Ser Gly Ser Thr Pro
            260

<210>  9
<211>  3876
<212>  DNA
<213>  Mus musculus

<220>
<221>  gene
<222>  (1)..(3876)
<223>  mouse PSMB5 gene ID19173

<400>  9
aaacatggcg ctggctagcg tgttgcagcg gccgatgccg gtgaatcagc acgggttttt      60

tgggctcgga ggtggtgcag atctgctgga cttgggtccg gggagtcctg gtgatgggct      120

gagcctagcc gcgccgagct ggggcgtccc ggaggagccg cgaatcgaaa tgcttcacgg      180

aaccaccacc ctggccttca aggtgctcag acagctcgcg cttctccggc atcccagccg      240

gaaccgggtc gggcggaggt tcggtcccct cctcccgacc cttggcgggt tcactgtctc      300

gtagccttaa gaaaggagag gtgggcggag atagggggcg gtgagggaag gagacggaca      360

gcgtgggttg gctcctaggc gaccccagtt tgcccctttt caggagccct acctgggaac      420

agcggaccgt ggcggagggt gcggggggga agccattgtc gccgttcacc aaagtttgta      480

taaactgagc ttgatgaagt ccaggggcta ctgaagtggg taccgaacag ttagggtccc      540

tgttccagcc ctacaataac tagctctgga tcttaggcta gtctttaaag atttattctt      600

attttttgta tatacaccac gtgtgtagtg ctcgcaggcg ccaaaagagg gcgtcgtgtt      660

ccttgggact ggagttacag acctttgtga gcactgggaa ccgaacctct gaagagcagc      720

cagtgctctt aaccactgag ctctctctcc agtcccctta gggtgtcttt atctcattaa      780

acttgctact tcatatttaa agtgagtaaa agggctattc ataaatcagg aactgctgtt      840

aaacccgaat gacatttaac ataagaagtc gattacgccg ggtggtggtg gctcacgcct      900

ttagtcccag cacatgggag gcagaggcag gtggatttct gagttcgagg ccagcctggt      960

ctacaaagtg agttccagga cagccaggac tatacagaga aaccctgtct cgaaaaacaa 1020

aaacaaaccc caaacccccc aaaaaagaag tcgattccaa aatcccaaaa gatgcaaagc 1080

caggttatct ttctgtattt atacaaaaat aaagtatggg gagtatttgt ggtcttacgg 1140

ggcctctctt tttgttttct ctgaccttaa ctagtttctc catggagtca ttgttgcagc 1200

ggattcccgg gccacagcag gtgcttatat tgcttcccag acggtgaaga aagtaataga 1260

gatcaacccg taccttctgg gcaccatggc tgggggtgca gcggattgca gcttctggga 1320

gcggttgttg gctcggcagt gtcgaatcta tgagcttcgc aataaggaac gcatctcggt 1380

cgcagcagcc tccaaactgc tcgctaacat ggtgtatcag tacaaaggca tggggctgtc 1440

tatgggcacc atgatctgtg gctgggataa gagaggccct ggtgagttgt gtcctgtgag 1500

ccatggtggg aaggggttaa atgggtggga gaattgaggg cttttaacag gtgctgggaa 1560

tggatgcgat attaggttct cagctcttcc ttccctttcc aaggaaggcc ttgtacggaa 1620

taggaaagag catagagtta gacttgctgt gtcactcact gcttagtcat ttgaccttag 1680

cctatttctt catctgggaa ctggttaagt gaaccattga agcggttaag tgtggtgcac 1740

atagagcaat tggtacaatg tctggcacaa cagctctcct tctttcccat gcaataatga 1800

ctagaaaaca tttgtactgg ggaattgtaa actttaaaaa ttgtgttcta ttctagccag 1860

gtgtggtggc acatgccttt aatcccaaca cttgggaggc agaggcaggc ggatttctga 1920

gttcgaggcc agcctggtct acaaagtgag ttccaggaca gccagggcta tacagagaaa 1980

ccctgtctca aaaaaccaaa agaaaaaaaa ttgtgttctt ttctatgctt gggtgttttg 2040

tctgcatgta tgactgtgca ccacagtgca ttcatggtgc ctgaagaggc cagagagggg 2100

attggatcct ctagaatgca gttactgatg gttgtgagcc acatgtgggt gctcagagct 2160

aaacccaggt ccttggcaag agtggcccgt ccttcttccc cctgccctgg gggccatctc 2220

cccagccccc cccccacttt taaattttaa gttaaattat ttatttgtgt gtgtatgtgt 2280

gaacaaacac atcctacagc atatctgcag ttcagaggat aacttgttca agttggttct 2340

cttgttctgt gggccgggct caggacatca ggcctggtgg taagcaacag ttatatgctg 2400

actcacctca ctggccctca ttgtaaaaaa taaataagcc aagtgataca cagctgtgac 2460

cctagagcac tagggagggt caagagttcg aggccagcct aagctgtatg gcagaaggac 2520

agcctggagc acacagaaac attgtatctc agaagtcagt aaaactagtg gagttggtgt 2580

atagttaatt ttatataaaa tgcccaaatt atttctcagt acctggcggt agtaccattt 2640

caagtgttga agtcagtttc atgcagtctc tagccactgt gttgcatgac aacgataggc 2700

tacagagttg tcatttaagc gttatctccc accattcctg agtcctgtgc ttgacttggc 2760

cagcatgctt gccttttgga gttgccgaga gagcaggagg gatggttaac atactgtgag 2820

gagagatggg tggtatggtg ctctggaatc tggaaatctc aagataaatt tgactctgtc 2880

tttctctgat ttcttgaaaa tttgtttctt tatgagtttc tgtatgggta tgtgagtgtt 2940

tgcatgtgta tatgtgtgta taccacttgc atgcctggtg cccacagagg acagaagaag 3000

gtattgcatc cccagagact ggagttacag acagttgcga gccaccccgt gtatattggg 3060

aactgaacct gggtcctaga caagagcaac aagcactctt aactgccgag gaagctttct 3120

agcccgtttt gttttgagac agggttttat gtagcccagg ctgacctcaa acttaataca 3180

tagtcaagcc tagcctttaa ctcctgatcc tcctgccttc acctcccaag tgtgctgaga 3240

tttgtttttg ttttgtttgt ttgtttgttt ggttggttgg ttggttggtt ttttgttttt 3300

tcgagacagg gtttctctgt gtagccctgg ctgtcctgga cctcactttg tagaccaggc 3360

tggcctcgta ctcagaaatc ctcctgtctc tgcctcccaa ggagatccta tttttgtcgg 3420

ggtggggcgg ggtggtgtgt gtgagagaga gagagaggga ggggtgttaa aatgacgtct 3480

tttttctcgt tccctgctca cctcatcccc tcaggcctct actacgtaga cagcgagggg 3540

aacaggatct ctgggaccgc cttctcagtg ggctctggct ccgtgtatgc ttacggcgtt 3600

atggatcgag gctactccta tgacctgaaa gtggaggagg cctatgatct ggcccgccga 3660

gccatctacc aagccaccta cagagatgcc tactccggag gggcagtcaa cctctaccac 3720

gtgcgggagg atggctggat ccgggtgtcc agtgacaatg tagctgactt acatgacaag 3780

tacagtagtg tatctgtccc ctgagggaag gtggatgcgg ttgtctgcat ctctaggtgg 3840

gtctccatgg ctataataaa taatagtacc tctttc 3876

<210> 10
<211> 21
<212> DNA
<213> Mus musculus

<220>
<221> primer_bind
<222> (1)..(21)
<223> msPSMB5exon2_fwd

<400> 10
gtatttgtgg tcttacgggg c 21

<210> 11
<211> 22
<212> DNA
<213> Mus musculus

<220>
<221> primer_bind
<222> (1)..(22)
<223> msPSMB5exon2_rev

<400>  11
aaccagttcc cagatgaaga aa                                                22

<210>  12
<211>  20
<212>  DNA
<213>  Mus musculus

<220>
<221>  gene
<222>  (1)..(20)
<223>  msPSMB5exon3_fwd

<400>  12
gggtggtgtg tgtgagagag                                                   20

<210>  13
<211>  20
<212>  DNA
<213>  Mus musculus

<220>
<221>  primer_bind
<222>  (1)..(20)
<223>  msPSMB5exon3_rev

<400>  13
ccagggttcg ggggagatat                                                   20

<210>  14
<211>  205
<212>  PRT
<213>  Mus musculus

<220>
<221>  PEPTIDE
<222>  (1)..(205)
<223>  mouse beta-5 peptide

<400>  14

Thr Thr Thr Leu Ala Phe Lys Phe Leu His Gly Val Ile Val Ala Ala
1               5                   10                  15

Asp Ser Arg Ala Thr Ala Gly Ala Tyr Ile Ala Ser Gln Thr Val Lys
            20                  25                  30

Lys Val Ile Glu Ile Asn Pro Tyr Leu Leu Gly Thr Met Ala Gly Gly
        35                  40                  45

Ala Ala Asp Cys Ser Phe Trp Glu Arg Leu Leu Ala Arg Gln Cys Arg
    50                  55                  60

Ile Tyr Glu Leu Arg Asn Lys Glu Arg Ile Ser Val Ala Ala Ala Ser
65                  70                  75                  80

Lys Leu Leu Ala Asn Met Val Tyr Gln Tyr Lys Gly Met Gly Leu Ser
                85                  90                  95

Met Gly Thr Met Ile Cys Gly Trp Asp Lys Arg Gly Pro Gly Leu Tyr
            100                 105                 110

Tyr Val Asp Ser Glu Gly Asn Arg Ile Ser Gly Thr Ala Phe Ser Val
        115                 120                 125

Gly Ser Gly Ser Val Tyr Ala Tyr Gly Val Met Asp Arg Gly Tyr Ser
    130                 135                 140

Tyr Asp Leu Lys Val Glu Glu Ala Tyr Asp Leu Ala Arg Arg Ala Ile
145                 150                 155                 160

Tyr Gln Ala Thr Tyr Arg Asp Ala Tyr Ser Gly Gly Ala Val Asn Leu
                165                 170                 175

Tyr His Val Arg Glu Asp Gly Trp Ile Arg Val Ser Ser Asp Asn Val
            180                 185                 190

Ala Asp Leu His Asp Lys Tyr Ser Ser Val Ser Val Pro
        195                 200                 205

<210>  15
<211>  264
<212>  PRT
<213>  Mus musculus

<220>
<221>  PROPEP
<222>  (1)..(264)
<223>  mouse beta-5 propolypeptide

<400>  15

Met Ala Leu Ala Ser Val Leu Gln Arg Pro Met Pro Val Asn Gln His
1               5                   10                  15

Gly Phe Phe Gly Leu Gly Gly Gly Ala Asp Leu Leu Asp Leu Gly Pro
            20                  25                  30

Gly Ser Pro Gly Asp Gly Leu Ser Leu Ala Ala Pro Ser Trp Gly Val
        35                  40                  45

Pro Glu Glu Pro Arg Ile Glu Met Leu His Gly Thr Thr Thr Leu Ala
    50                  55                  60

Phe Lys Phe Leu His Gly Val Ile Val Ala Ala Asp Ser Arg Ala Thr
65                  70                  75                  80

Ala Gly Ala Tyr Ile Ala Ser Gln Thr Val Lys Lys Val Ile Glu Ile
                85                  90                  95

Asn Pro Tyr Leu Leu Gly Thr Met Ala Gly Gly Ala Ala Asp Cys Ser
            100                 105                 110

Phe Trp Glu Arg Leu Leu Ala Arg Gln Cys Arg Ile Tyr Glu Leu Arg
        115                 120                 125

Asn Lys Glu Arg Ile Ser Val Ala Ala Ala Ser Lys Leu Leu Ala Asn
    130                 135                 140

Met Val Tyr Gln Tyr Lys Gly Met Gly Leu Ser Met Gly Thr Met Ile
145                 150                 155                 160

Cys Gly Trp Asp Lys Arg Gly Pro Gly Leu Tyr Tyr Val Asp Ser Glu
                165                 170                 175

Gly Asn Arg Ile Ser Gly Thr Ala Phe Ser Val Gly Ser Gly Ser Val
            180                 185                 190

Tyr Ala Tyr Gly Val Met Asp Arg Gly Tyr Ser Tyr Asp Leu Lys Val
        195                 200                 205

Glu Glu Ala Tyr Asp Leu Ala Arg Arg Ala Ile Tyr Gln Ala Thr Tyr
    210                 215                 220

Arg Asp Ala Tyr Ser Gly Gly Ala Val Asn Leu Tyr His Val Arg Glu
225                 230                 235                 240

Asp Gly Trp Ile Arg Val Ser Ser Asp Asn Val Ala Asp Leu His Asp
                245                 250                 255

Lys Tyr Ser Ser Val Ser Val Pro
            260

## Description

### Field of the Invention

The present application relates to an *in vitro* method and a diagnostic kit for identifying resistance to bortezomib treatment in a patient by detecting one or more substitutions in the amino acid sequence of the polypeptide β5 (PSMB5). The present application also relates to a composition and preferably a synergistic composition of bortezomib and a further drug suitable for the treatment of a patient suffering from a disease selected from the group comprising multiple myeloma, mantle cell lymphoma, non-Hodgkin's lymphoma, TEMPI syndrome, light chain deposition disease (LCDD), IgG₄-related disease, and scleromyxedema.

### Background of the invention

Multiple myeloma (MM) is an incurable disease that is characterized by clonal expansion of malignant plasma cells in the bone marrow (Gandolfi S et al., Cancer Metastasis Rev. 2017, 36, 561-584). This cell proliferation causes osteolytic bone lesions, which are oftentimes accompanied by severe hypercalcemia. Secondary consequences of monoclonal immunoglobulin production by malignant plasma cells include kidney failure and blood hyperviscosity. All of these complications contribute to the high disease burden associated with multiple myeloma. In 2018, the worldwide incidence of multiple myeloma was estimated at 159,958 cases and the disease led to approximately 106,000 deaths, comprising 1.1% of all cancer-associated mortality. Due to major advances in multiple myeloma treatment, overall survival has improved substantially in the last two decades. Numerous novel compounds have been developed, allowing for successful treatment with acceptable side effects even in elderly and multimorbid patients for whom autologous stem cell transplantation is not a viable option. However, most patients eventually relapse and the period of remission decreases with each iteration of therapy. Optimal use of existing drugs is therefore of utmost priority.

Blood cancers encompassing multiple myeloma (MM), and certain Non-Hodgkin's lymphomas (NHL) such as mantle cell lymphoma (MCL), have been found to rely on optimal performance of the ubiquitin-proteasomal degradation system (UPS). In comparison to normal, the malignantly transformed lymphocytes have a significantly higher protein turnover rate and therefore are highly sensitive to proteasome inhibitors (PI) such as bortezomib (e.g. VELCADE®) or carfilzomib (e.g. KYPROLIS®).

Proteasome inhibitors are applied throughout the different treatment phases and are considered a first-line option for many patients, frequently in combination with immunomodulatory and antiproliferative drugs (Gandolfi S et al., Cancer Metastasis Rev. 2017, 36, 561-584).
Bortezomib, the first proteasome inhibitor approved for multiple myeloma treatment, is a dipeptide boronic acid that targets the proteasome subunit β5, encoded by the PSMB5 gene. The β5 subunit is a component of the essential 20S core proteasome complex that possesses the chymotrypsin-like proteolytic activity. In addition to the β5 subunit, bortezomib also binds to β1 (caspase-like activity) and β2 (trypsin-like activity), albeit to a much lesser extent. Upon exposure to proinflammatory cytokines, the constitutive proteasome subunits β1, β2, and β5 are replaced by their counterparts β1i, β2i, and β5i (LMP7/PSMB8), forming the immunoproteasome. Although bortezomib can bind both β5 and β5i, resistance-associated mutations have so far been observed only in the constitutive β5 proteasomal subunit. Importantly, somatic PSMB5 substitutions were recently identified in a bortezomib treated multiple myeloma patient, suggesting that resistance through PSMB5 point mutations is a clinically relevant mechanism.

Thus, it is the objective of the present invention to provide an *in vitro* method to identify PSMB5 mutations resulting in resistance to bortezomib and other proteasome inhibitors, as well as a diagnostic kit to perform this method.

This objective is solved by the teaching of the independent claims. Further advantageous features, aspects and details of the invention are evident from the dependent claims, the description, the figures, and the examples of the present application.

### Brief description of the invention

The application is directed to an *in vitro* method for identifying resistance to bortezomib treatment in a patient prior, during or after bortezomib treatment, comprising:
a) obtaining a biological sample containing diseased cells from said patient, and
b) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

Thus, the invention relates to a method, especially an *in vitro* method for identifying resistance to bortezomib in a patient in need thereof, which comprises detecting in the amino acid sequence of the polypeptide β5 one or more substitutions which inhibit the binding of bortezomib to the active site of the polypeptide β5, thereby identifying said patient as being resistant to bortezomib treatment.
In accordance with the invention, the identification of a resistance to bortezomib involves the detection of one or more mutations in the PSMB5 gene encoding the polypeptide β5, that result in one or more substitutions especially in the amino acid residues A22, V31, S130, Y169, and optionally in R19, A20, T21, A27, M45, A49, A50, C52, C63 and G183.

In general the method comprises or consists essentially of the steps (a) obtaining a biological sample containing diseased cells from said patient, (b) detecting a substitution in the amino acid sequence of the polypeptide β5 in at least one of the amino acid residues A22, V31, S130, Y169, and optionally R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

The term "detecting a substitution in the amino acid sequence of the polypeptide β5" refers preferably to the detection of a mutation in the nucleic acid sequence of the PSMB5 gene which leads to the corresponding amino acid substitution in the amino acid sequence of the polypeptide β5.

The biological sample containing diseased cells of step (a) can be obtained from bone marrow, whole blood, mononuclear cells, or plasma cells from said patient. For instance, the invention relates to a method for identifying resistance to bortezomib in a patient suffering from multiple myeloma, mantle cell lymphoma, non-Hodgkin's lymphoma, TEMPI syndrome, light chain deposition disease (LCDD), IgG₄-related disease, or scleromyxedema.

More specifically, the substitution in the amino acid sequence of the polypeptide β5 can be detected by (a') determining the nucleic acid sequence of the PSMB5 gene encoding the polypeptide β5, and (a") detecting a mutation in the nucleic acid sequence of the PSMB5 gene that results in a substitution in the amino acid sequence of the polypeptide β5 especially in at least one of the amino acid residues A22, V31, S130, Y169, and optionally R19, A20, T21, A27, M45, A49, A50, C52, C63 and G183. For instance, determining the nucleic acid sequence of the PSMB5 gene can comprise DNA sequencing.

The invention refers further to a composition and preferably a synergistic composition of bortezomib and at least one further drug suitable for the treatment of a patient in need thereof, wherein a substitution in the amino acid sequence of the polypeptide β5 in at least one of the amino acid residues A22, V31, S130, Y169, and optionally R19, A20, T21, A27, M45, A49, A50, C52, C63, and G183 does not result in resistance to that further drug. The further drug can be a second generation proteasome inhibitor, such as carfilzomib (PR-171), oprozomib (ONX-0912), ixazomib (MLN9708 / MLN2238), delanzomib (CEP-18770), and marizomib (NPI-0052), or Syringolins and Syringolin analogues. Alternatively, the further drug can be an immunomodulatory compound, such as lenalidomide, pomalidomide, or thalidomide. In another alternative, the further drug can be an antiproliferative drug such as melphalan, vincristine, doxorubicin, bendamustine und cyclophosphamide. In another alternative, the further drug can be a monoclonal antibody such as anti-IL6, anti-CXC4, anti-CD38, or the anti SLAMF7 antibody elotozumab. In another alternative, the further drug can be a histone deacetylase (HDAC) inhibitor.

The invention also provides a diagnostic kit for identifying resistance to bortezomib in a patient, comprising an extraction system to isolate RNA or genomic DNA (gDNA), and sensitive oligonucleotide primer pairs to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues A22, V31, S130, Y169, and optionally R19, A20, T21, A27, M45, A49, A50, C52, C63, and G183. Thus, the kit also includes a means for revealing the polymerase chain reaction amplification of the PSMB5 gene. Another embodiment of the present invention refers to the use of said diagnostic kit for providing an alternative treatment to patients with a resistance to bortezomib treatment.

### Detailed description of the invention

Bortezomib was the first proteasome inhibitor discovered and demonstrated great efficacy in myeloma, both *in vitro* and in patients. Therefore, bortezomib has now become a first-line treatment for many patients, showing better response rates than previous regimens. However, relapse is common in multiple myeloma even when using maintenance therapy.

Thus, while treatment of relapsed multiple myeloma with second-generation proteasome inhibitors can lead to remission, there is a significant risk for broad resistance leading to the choice of ineffective regimens that come along with substantial side effects. Due to limited knowledge regarding shared and distinct resistance mechanisms between different proteasome inhibitors, there is no established protocol for rational stratification of multiple myeloma patients available at this time.

The present application discloses an *in vitro* method for analysing whether a patient has or has developed a bortezomib resistance. This *in vitro* method for identifying resistance to bortezomib treatment in a patient prior, during or after bortezomib treatment, comprises:
a) obtaining a biological sample containing diseased cells from said patient, and
b) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

In simpler words, the present application is directed to an *in vitro* method for identifying resistance to bortezomib treatment in a patient prior, during or after bortezomib treatment, comprising:
a) obtaining a biological sample containing diseased cells from said patient, and
b) detecting a substitution in the amino acid sequence of the polypeptide β5 (PSMB5) in at least one of the amino acid residues A22, V31, S130 and Y169.

In a preferred embodiment of the invention, the *in vitro* method for identifying resistance to bortezomib treatment in a patient, further comprises at step (b) detecting a substitution in the amino acid sequence of the polypeptide β5 in at least one of the amino acid residues R19, A20, T21, A27, M45, A49, A50, C52, C63, and G183.

Therefore, the present invention is also directed to an *in vitro* method for identifying resistance to bortezomib treatment in a patient prior, during or after bortezomib treatment, comprising:
a) obtaining a biological sample containing diseased cells from said patient, and
b) detecting a substitution in the amino acid sequence of the polypeptide β5 (PSMB5) in at least one of the amino acid residues A22, V31, S130, and Y169, and detecting a substitution in the amino acid sequence of the polypeptide β5 (PSMB5) in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

In simpler words, the present invention is directed to an *in vitro* method for identifying resistance to bortezomib treatment in a patient prior, during or after bortezomib treatment, comprising:
a) obtaining a biological sample containing diseases cells from said patient, and
b) detecting a substitution in the amino acid sequence of the polypeptide β5 (PSMB5) in at least one of the amino acid residues A22, V31, S130, and Y169, and optionally detecting a substitution in at least one of the amino acid residues R19, A20, T21, A27, M45, A49, A50, C52, C63, and G183.

More specifically, the biological sample containing diseased cells in step (a) of the inventive methods is preferably obtained from bone marrow, whole blood, mononuclear cells, or plasma cells.

Thus, the present invention is directed to an *in vitro* method for identifying resistance to bortezomib treatment in a patient prior, during or after bortezomib treatment, comprising:
a) obtaining a biological sample containing diseased cells from bone marrow, whole blood, mononuclear cells, or plasma cells from said patient, and
b) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

In still another embodiment, the present invention is directed to an *in vitro* method for identifying resistance to bortezomib treatment in a patient prior, during or after bortezomib treatment, comprising:
a) obtaining a biological sample containing diseased cells from bone marrow, whole blood, mononuclear cells, or plasma cells from said patient, and
b) detecting a substitution in the amino acid sequence of the polypeptide β5 (PSMB5) in at least one of the amino acid residues A22, V31, S130, and Y169, and optionally detecting a substitution in the amino acid sequence of the polypeptide β5 (PSMB5) in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

### Diseases treated with Bortezomib

The term **"bortezomib"** as used herein refers to a dipeptide boronoic acid analogue having the chemical structure name [(1R)-3-Methyl-1-[[(2S)-1-oxo-3-phenyl-2-[(pyrazinylcarbonyl)amino]propyl]amino]butyl]boronic acid and includes all pharmaceutically acceptable salts, solvates, and prodrugs thereof. Bortezomib is also called PS-341 and is also known by the brand name VELCADE®. Bortezomib reversibly and competitively inhibits the 26S proteasome.

The term **"prodrug"** as used herein refers preferably to MG132 and the compound class MG132 belongs to. MG132 is a tripeptide of three leucine amino acids and belongs to the compound class of oligopeptides with two to five amino acids, wherein the majority or all amino acids are leucine and wherein the other amino acids, if present, are lipophilic amino acids such as glycine, alanine, cysteine, isoleucine, methionine, phenylalanine, and proline. MG132 has the structure as shown below:

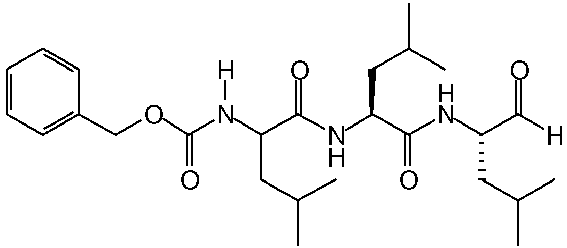

The term **"hematological malignancy"** or **"hematologic malignancy"** as used herein refers to a cancer that affects blood and bone marrow.

The term **"leukemia"** as used herein means any disease involving the progressive proliferation of abnormal leukocytes found in hemopoietic tissues, other organs and usually in the blood in increased numbers. For example, leukemia includes acute myeloid leukemia, acute lymphocytic leukemia and chronic myeloma leukemia (CML) in blast crisis.

The term **"lymphoma"** as used herein means any disease involving the progressive proliferation of abnormal lymphoid cells. For example, lymphoma includes mantle cell lymphoma, non-Hodgkin's lymphoma, and Hodgkin's lymphoma. **Non-Hodgkin's lymphoma** would include indolent and aggressive Non-Hodgkin's lymphoma. Aggressive Non-Hodgkin's lymphoma would include intermediate and high grade lymphoma. Indolent Non-Hodgkin's lymphoma would include low grade lymphomas. The **mantle cell lymphoma** is also an example of an aggressive, non-Hodgkins lymphoma. Mantle cell lymphoma is found in lymph nodes, the spleen, bone marrow, blood, and sometimes the gastrointestinal system (lymphomatous polyposis). Like the low-grade lymphomas, mantle cell lymphoma appears incurable with anthracycline-based chemotherapy and occurs in older patients with generally asymptomatic advanced-stage disease. However, the median survival is significantly shorter (3-5 years) than that of other lymphomas; hence this histology is now considered to be an aggressive lymphoma.

The term **"myeloma"** and/or **"multiple myeloma"** as used herein means any tumor or cancer composed of cells derived from the hemopoietic tissues of the bone marrow. Multiple myeloma is also knows as MM and/or plasma cell myeloma.

**"TEMPI syndrome"** is an orphan disease where the person share five characteristics from which the acronym is derived: Telangiectasias, Elevated Erythropoietin and Erythrocytosis, Monoclonal gammopathy, Perinephric fluid collection, and Intrapulmonary shunting.

**"Light-chain deposition disease (LCDD)"** is a deposition of monoclonal, amorphous, noncongophilic light chains in multiple organs that do not typically exhibit a fibrillar structure when examined ultrastructurally. LCDD is an infiltration of light chains involving multiple organs. Renal disease, including renal insufficiency, proteinuria, and nephrotic syndrome, is the major manifestation of LCDD. Many cases are associated with multiple myeloma or lymphoproliferative disease, but as many as 50% of patients have no evidence of neoplastic plasma cell proliferation.

**"IgG₄-related disease"** (IgG₄-RD) is unique clinical condition where an inflammatory lesion closely resembles a tumor and hence is referred to as a pseudotumorous or a tumefactive lesion. IgG4-related disease is recognized now as a unique clinicopathologic entity characterized by tumefactive, fibroinflammatory lesions, the infiltration of IgG₄-positive plasma cells into affected tissues, and often elevated concentrations of IgG4 in serum. The most common gastrointestinal manifestations include autoimmune pancreatitis and IgG₄-related sclerosing cholangitis. Although the diagnosis of IgG4-related disease is based on a constellation of clinical, radiological, and pathologic findings, histopathology is the gold standard for diagnosis.

**"Scleromyxedema"** is a rare, severe skin disorder. Signs and symptoms include abnormal accumulation of mucin in the skin (mucinosis), causing papular and sclerodermoid bumps; increased production of fibroblasts (connective tissue cells) in the absence of a thyroid disorder; and monoclonal gammopathy (abnormal proteins in the blood). It often involves internal organs and may affect various body systems. The cause of scleromyxedema is not known. In a few cases, it has been reported in association with cancers of the bone marrow such as myeloma, lymphoma and leukemia. There is no standard treatment. Management may involve the use of intravenous immunoglobulin, plasmapheresis, thalidomide and corticoids. When the patients with severe disease do not have a good response, other interventions are required, such as autologous bone marrow transplantation, melphalan, or bortezomib with dexamethasone.

As used herein, the terms **"subject", "individual"** and **"patient"** are used interchangeably. None of the terms are to be interpreted as requiring the supervision of a medical professional (e.g., a doctor, nurse, physician's assistant, orderly, hospice worker). As used herein, the subject can be any animal, including mammals (e.g., a human or non-human animal) and non-mammals. In a preferred embodiment of the invention provided herein, the mammal is a human.

Bortezomib is the treatment of first choice for multiple myeloma. Recent studies show that bortezomib could also be used to treat other diseases such as mantle cell lymphoma, non-Hodgkin's lymphoma, TEMPI syndrome, light chain deposition disease (LCDD), IgG₄-related disease, or scleromyxedema.

As a consequence, the present invention relates to an *in vitro* method, wherein the patient is suffering from a disease selected from the group comprising or consisting of multiple myeloma, mantle cell lymphoma, non-Hodgkin's lymphoma, TEMPI syndrome, light chain deposition disease (LCDD), IgG₄-related disease, or scleromyxedema.

Consequently, the present invention refers particularly to an *in vitro* method for identifying resistance to bortezomib treatment in a patient suffering from a disease selected from the group comprising or consisting of multiple myeloma, mantle cell lymphoma, non-Hodgkin's lymphoma, TEMPI syndrome, light chain deposition disease (LCDD), IgG₄-related disease, or scleromyxedema, prior, during or after bortezomib treatment, comprising:
a) obtaining a biological sample containing diseased cells from said patient, and
b) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

In another embodiment, the present invention refers to an *in vitro* method for identifying resistance to bortezomib treatment in a patient suffering from a disease selected from the group comprising or consisting of multiple myeloma, mantle cell lymphoma, non-Hodgkin's lymphoma, TEMPI syndrome, light chain deposition disease (LCDD), IgG₄-related disease, or scleromyxedema, prior, during or after bortezomib treatment, comprising:
a) obtaining a biological sample containing diseased cells from said patient, and
b) detecting a substitution in the amino acid sequence of the polypeptide β5 (PSMB5) in at least one of the amino acid residues A22, V31, S130, and Y169, and optionally detecting a substitution in the amino acid sequence of the polypeptide β5 (PSMB5) in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

In particular, the present invention refers to an *in vitro* method for identifying resistance to bortezomib treatment in a patient suffering from a disease selected from the group comprising or consisting of multiple myeloma, mantle cell lymphoma, non-Hodgkin's lymphoma, TEMPI syndrome, light chain deposition disease (LCDD), IgG₄-related disease, or scleromyxedema, prior, during or after bortezomib treatment, comprising:
a) obtaining a biological sample containing diseased cells from bone marrow, whole blood, mononuclear cells, or plasma cells from said patient, and
b) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

In still another instance, the present invention refers to an *in vitro* method for identifying resistance to bortezomib treatment in a patient suffering from a disease selected from the group comprising or consisting of multiple myeloma, mantle cell lymphoma, non-Hodgkin's lymphoma, TEMPI syndrome, light chain deposition disease (LCDD), IgG₄-related disease, or scleromyxedema, prior, during or after bortezomib treatment, comprising:
a) obtaining a biological sample containing diseased cells from bone marrow, whole blood, mononuclear cells, or plasma cells from said patient, and
b) detecting a substitution in the amino acid sequence of the polypeptide β5 (PSMB5) in at least one of the amino acid residues A22, V31, S130, and Y169, and optionally detecting a substitution in the amino acid sequence of the polypeptide β5 (PSMB5) in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

The present application relates also to an *in vitro* method for identifying resistance to bortezomib treatment in a patient suffering from a proteasome inhibitor sensitive disease prior, during or after bortezomib treatment, comprising:
a) obtaining a biological sample containing diseased cells from said patient, and
b) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

Moreover, the present application relates to an *in vitro* method for identifying resistance to bortezomib treatment in a patient suffering from a proteasome inhibitor sensitive disease disease prior, during or after bortezomib treatment, comprising:
a) obtaining a biological sample containing diseased cells from said patient, and
b) detecting a substitution in the amino acid sequence of the polypeptide β5 (PSMB5) in at least one of the amino acid residues A22, V31, S130, and Y169, and optionally detecting a substitution in the amino acid sequence of the polypeptide β5 (PSMB5) in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

Preferably after step a) and before step b) the following two steps can be introduced:
a') determining the nucleic acid sequence of the PSMB5 gene encoding the polypeptide β5, and
a") detecting a mutation in the nucleic acid sequence of the PSMB5 gene that results in a substitution in the amino acid sequence of the polypeptide β5 in at least one of the amino acid residues A22, V31, S130 and Y169.

Consequently, the present application is directed to an *in vitro* method for identifying resistance to bortezomib treatment in a patient prior, during or after bortezomib treatment comprising the steps:
a) obtaining a biological sample containing diseased cells from said patient,
   a') determining the nucleic acid sequence of the PSMB5 gene encoding the polypeptide β5,
   a") detecting a mutation in the nucleic acid sequence of the PSMB5 gene that results in a substitution in the amino acid sequence of the polypeptide β5 in at least one of the amino acid residues A22, V31, S130 and Y169, and
b) detecting or thereby detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

In another preferred embodiment, the present application is directed to an *in vitro* method for identifying resistance to bortezomib treatment in a patient prior, during or after bortezomib treatment comprising the steps:
a) obtaining a biological sample containing diseased cells from said patient, and
   a') determining the nucleic acid sequence of the PSMB5 gene encoding the polypeptide β5, and
   a") detecting a mutation in the nucleic acid sequence of the PSMB5 gene that results in a substitution in the amino acid sequence of the polypeptide β5 in at least one of the amino acid residues A22, V31, S130, and Y169, and optionally detecting a mutation in the nucleic acid sequence of the PSMB5 gene that results in a substitution in the amino acid sequence of the polypeptide β5 in at least one of the amino acid residues R19, A20, T21, A27, M45, A49, A50, C52, C63, and G183,
b) detecting or thereby detecting a substitution in the amino acid sequence of the polypeptide β5 in at least one of the amino acid residues A22, V31, S130, and Y169, and optionally detecting or thereby detecting a substitution in the amino acid sequence of the polypeptide β5 in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

Moreover, the present application is directed to an *in vitro* method for identifying resistance to bortezomib treatment in a patient prior, during or after bortezomib treatment comprising the steps:
a) obtaining a biological sample containing diseased cells from bone marrow, whole blood, mononuclear cells, or plasma cells from said patient, and
   a') determining the nucleic acid sequence of the PSMB5 gene encoding the polypeptide β5,
   a") detecting a mutation in the nucleic acid sequence of the PSMB5 gene that results in a substitution in the amino acid sequence of the polypeptide β5 in at least one of the amino acid residues A22, V31, S130 and Y169, and
b) detecting or thereby detecting a substitution in the amino acid sequence of the polypeptide β5 in at least one of the amino acid residues A22, V31, S130 and Y169, wherein said substitution inhibits the binding of bortezomib to the active site of the polypeptide β5, thereby identifying said patient as being resistant to bortezomib treatment.

Furthermore, the present application is directed to an *in vitro* method for identifying resistance to bortezomib treatment in a patient prior, during or after bortezomib treatment comprising the steps:
a) obtaining a biological sample containing diseased cells from bone marrow, whole blood, mononuclear cells, or plasma cells from said patient, and
   a') determining the nucleic acid sequence of the PSMB5 gene encoding the polypeptide β5, and
   a") detecting a mutation in the nucleic acid sequence of the PSMB5 gene that results in a substitution in the amino acid sequence of the polypeptide β5 in at least one of the amino acid residues A22, V31, S130, and Y169, and optionally detecting a mutation in the nucleic acid sequence of the PSMB5 gene that results in a substitution in the amino acid sequence of the polypeptide β5 in at least one of the amino acid residues R19, A20, T21, A27, M45, A49, A50, C52, C63, and G183,
b) detecting or thereby detecting a substitution in the amino acid sequence of the polypeptide β5 in at least one of the amino acid residues A22, V31, S130, and Y169, and optionally detecting or thereby detecting a substitution in the amino acid sequence of the polypeptide β5 (PSMB5) in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

Moreover, the present invention especially relates to an *in vitro* method for identifying resistance to bortezomib treatment in a patient suffering from a disease selected from the group comprising or consisting of multiple myeloma, mantle cell lymphoma, non-Hodgkin's lymphoma, TEMPI syndrome, light chain deposition disease (LCDD), IgG₄-related disease, or scleromyxedema, prior, during or after bortezomib treatment, comprising the steps:
a) obtaining a biological sample containing diseased cells from said patient, or
a) obtaining a biological sample containing diseased cells from bone marrow, whole blood, mononuclear cells, or plasma cells from said patient, and
   a') determining the nucleic acid sequence of the PSMB5 gene encoding the polypeptide β5, and
   a") detecting a mutation in the nucleic acid sequence of the PSMB5 gene that results in a substitution in the amino acid sequence of the polypeptide β5 in at least one of the amino acid residues A22, V31, S130 and Y169, and
b) detecting or thereby detecting a substitution in the amino acid sequence of the polypeptide β5 in at least one of the amino acid residues A22, V31, S130 and Y169, wherein said substitution inhibits the binding of bortezomib to the active site of the polypeptide β5, thereby identifying said patient as being resistant to bortezomib treatment.

In another preferred embodiment, the present invention refers to an *in vitro* method for identifying resistance to bortezomib treatment in a patient suffering from a disease selected from the group comprising or consisting of multiple myeloma, mantle cell lymphoma, non-Hodgkin's lymphoma, TEMPI syndrome, light chain deposition disease (LCDD), IgG₄-related disease, or scleromyxedema, prior, during or after bortezomib treatment, comprising the steps:
a) obtaining a biological sample containing diseased cells from said patient, or
a) obtaining a biological sample containing diseased cells from bone marrow, whole blood, mononuclear cells, plasma cells from said patient, and
   a') determining the nucleic acid sequence of the PSMB5 gene encoding the polypeptide β5, and
   a") detecting a mutation in the nucleic acid sequence of the PSMB5 gene that results in a substitution in the amino acid sequence of the polypeptide β5 in at least one of the amino acid residues A22, V31, S130, and Y169, and optionally detecting a mutation in the nucleic acid sequence of the PSMB5 gene that results in a substitution in the amino acid sequence of the polypeptide β5 in at least one of the amino acid residues R19, A20, T21, A27, M45, A49, A50, C52, C63, and G183, and
b) detecting or thereby detecting a substitution in the amino acid sequence of the polypeptide β5 in at least one of the amino acid residues A22, V31, S130, and Y169, and optionally detecting or thereby detecting a substitution in the amino acid sequence of the polypeptide β5 (PSMB5) in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

### Gene, proteins , mutations, substitutions

The term **"gene"** refers to a nucleic acid comprising an open reading frame encoding a polypeptide, including both exon and (optionally) intron sequences. The nucleic acid may also optionally include non-coding sequences such as promoter or enhancer sequences. The term "intron" refers to a DNA sequence present in a given gene that is not translated into protein and is generally found between "exons".

A **"protein"** is a polypeptide that performs a structural or functional role in a living cell.

The term **"amino acid"** refers to naturally occurring and non-naturally occurring amino acids, as well as amino acid analogs and amino acid mimetics that function in a manner similar to the naturally occurring amino acids. Naturally encoded amino acids are the 20 common amino acids (alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine) and pyrolysine and selenocysteine. Amino acid analogs refers to agents that have the same basic chemical structure as a naturally occurring amino acid, i.e., an a carbon that is bound to a hydrogen, a carboxyl group, an amino group, and an R group, such as, homoserine, norleucine, methionine sulfoxide, methionine methyl sulfonium. Such analogs have modified R groups (such as, norleucine) or modified peptide backbones, but retain the same basic chemical structure as a naturally occurring amino acid.

**"Amino acids"** are referred to herein by either their commonly known three letter symbols or by the one-letter symbols recommended by the IUPAC-IUB Biochemical Nomenclature Commission. **"Nucleotides",** likewise, are referred to by their commonly accepted single-letter codes.

The terms **"amino acid"** and **"amino acid sequence"** refer to an oligopeptide, peptide, polypeptide, or protein sequence (which terms may be used interchangeably), or a fragment of any of these, and to naturally occurring or synthetic molecules. Where "amino acid sequence" is recited to refers to a sequence of a naturally occurring protein molecule, "amino acid sequence" and like terms are not meant to limit the amino acid sequence to the complete native amino acid sequence associated with the recited protein molecule.

The terms **"polypeptide", peptide"** and **"protein"** are used interchangeably herein to refer to a polymer of amino acid residues. The terms apply to naturally occurring amino acid polymers as well as amino acid polymers in which one or more amino acid residues is a non-naturally occurring amino acid, e.g., an amino acid analog or a modified amino acid. The terms encompass amino acid sequences of any length, including full length proteins, wherein the amino acid residues are linked by covalent peptide bonds.

As used herein, a **"modification"** is in reference to a modification of a sequence of amino acids of a polypeptide or of a sequence of nucleotides in a nucleic acid molecule and includes deletions, insertions, and substitutions of amino acids or nucleotides, respectively.

In some embodiments, the altered amino acid sequence is at least 75% identical, e.g., 77%, 80%, 82%, 85%, 88%, 90%, 91%, 92%, 93%, 94%, 95%, 96%, 97%, 98%, 99%, or 100% identical to the amino acid sequence of the wild-type protein described herein. Such sequence-variant proteins are suitable for the methods described herein as long as the altered amino acid sequence retains sufficient biological activity to be functional in the compositions and methods described herein.

The gene with a mutation may encode a modified amino acid sequence. In this regard, the mutation may be a "non-silent mutation". Non-limiting examples of nucleic acid mutations that may be detected in the inventive methods include missense, nonsense, insertion, deletion, duplication, frameshift, and repeat expansion mutations. The mutation may be a loss-of- function mutation. A loss-of-function mutation decreases or eliminates expression or activity of the encoded polypeptide. One or more mutations may be detected in a given gene in accordance with the invention. According to a preferred embodiment of the invention, a mutation in the PSMB5 gene sequence results in an amino acid substitution in the polypeptide β5, which can inhibit the binding of bortezomib to the active site of the polypeptide β5.

A **"substitution"** or **"replacement"** are used interchangeably herein and refer to a change in the amino acid or nucleotide sequence that results in the substitution of one or more amino acid residues or nucleotides. A substitution may include an internal substitution or a terminal substitution.

A **"variant," "mutant", or "derivative"** of a particular nucleic acid sequence may be defined as a nucleic acid sequence having at least 50% sequence identity to the particular nucleic acid sequence over a certain length of one of the nucleic acid sequences using blastn with the "BLAST 2 Sequences" tool available at the NCBI website. (Tatusova A. et al., FEMS Microbiol Lett. 1999, 174:247-250). Such a pair of nucleic acids may show, for example, at least 60%, at least 70%, at least 80%, at least 85%, at least 90%, at least 91%, at least 92%, at least 93%, at least 94%, at least 95%, at least 96%, at least 97%, at least 98%, or at least 99% or greater sequence identity over a certain defined length. Nucleic acid sequences that do not show a high degree of identity may nevertheless encode similar amino acid sequences due to the degeneracy of the genetic code. It is understood that changes in a nucleic acid sequence can be made using this degeneracy to produce multiple nucleic acid sequences that all encode substantially the same protein.
**Changes or differences** in nucleotide sequence between closely related nucleic acid sequences may arise during the course of normal replication or duplication in nature of the particular nucleic acid sequence. Other changes may be specifically designed and introduced into the sequence for specific purposes, such as to change an amino acid codon or sequence in a region of interest of the nucleic acid. Such specific changes may be made in vitro using a variety of mutagenesis techniques (e.g. **site-directed mutagenesis described** by Wang et al., 2011) or produced in a host organism placed under particular selection conditions that induce or select for the changes. Such sequence variants generated specifically may also be referred to as **"variants", "mutants"** or **"derivatives"** of the original sequence.

A **"variant," "mutant," or "derivative"** of a particular polypeptide sequence is defined as a polypeptide sequence having at least 50% sequence identity to the particular polypeptide sequence over a certain length of one of the polypeptide sequences using blastp with the "BLAST 2 Sequences" tool available at the NCBI website. (Tatusova A. et al., FEMS Microbiol Lett. 1999, 174:247-250). Such a pair of polypeptides may show, for example, at least 60%, at least 70%, at least 80%, at least 90%, at least 91%, at least 92%, at least 93%, at least 94%, at least 95%, at least 96%, at least 97%, at least 98%, or at least 99% or greater sequence identity over a certain defined length of one of the polypeptides. A **"variant"** or a **"mutant"** may have substantially the same functional activity as a reference polypeptide. For example, a variant or mutant of a chymotrypsin-like protease or catalytic subunit may have chymotrypsin-like activity

The term **"amino acid residues** A22, V31, S130, Y169, R19, A20, T21, A27, M45, A49, A50, C52, C63, and G183 in the amino acid sequence of the polypeptide β5 " as used herein means an amino acid corresponding in terms of position and/or function to the before listed amino acids in the polypeptide β5, which is a component of the mammalian 20S proteasome (e.g. human, mouse) and has chymotrypsin-like activity. A person skilled in the art would be familiar with methodologies for identifying corresponding amino acids, for example using sequence alignment, structured alignment etc.

The **"β5 polypeptide"** is also known as 20S proteasome subunit beta-5. This protein is one of the 17 essential subunits that contribute to the complete assembly of 20S proteasome complex. This β5 polypeptide possesses "chymotrypsin-like" activity and is capable of cleaving after large hydrophobic residues of peptide. The β5 polypeptide is originally expressed as a precursor with 263 amino acids. The propeptide fragment of 59 amino acids at N-terminal is essential for proper protein folding and subsequent complex assembly. At the end-stage of complex assembly, the N-terminal propeptide is cleaved, forming the mature β5 polypeptide of the 20S complex 204 amino acids long (SEQ ID No. 9). As disclosed herein the **β5 polypeptide** refers to the mature form of 204 amino acids.

The **"proteasome"** as used herein refers to a multimeric enzymatic complex involved in the degradation of protein and includes a 20S particle and two 19S components which together form the 26S proteasome. The 20S particle consists of four stacked heptameric ring structures that are themselves composed of two different types of subunits; α subunits which are structural in nature and β subunits which are predominantly catalytic. The proteasome comprises multiple protease activities including a chymotrypsin-like protease activity. The proteasomal degradation pathway is necessary to rid cells of excess and misfolded proteins as well as to regulate levels of proteins responsible for processes such as cell cycle progression, DNA repair and transcription.

### Modified β5 polypeptides or variants.

Provided herein are modified β5 polypeptides, also referred to as β5 polypeptides variants. In some embodiments, the modified β5 polypeptides are isolated modified β5 polypeptides. In some embodiments, the isolated modified β5 polypeptides are non-native modified β5 polypeptides. In some embodiments, the modified β5 polypeptides are recombinant proteins. In some embodiments, the modified β5 polypeptides are purified from a host cell. In some embodiments, the modified β5 polypeptides comprise one or more substitutions. In some embodiments, one or more substitutions in the modified β5 polypeptides result in resistance of a patient to treatment with bortezomib. In some embodiments, the one or more substitutions result in inhibition of the binding of bortezomib to the active site of the polypeptide β5.

In some embodiments, the one or more substitutions result in a modification at an amino acid position corresponding to the amino acid residue A22, V31, S130, Y169, and optionally R19, A20, T21, A27, M45, A49, A50, C52, C63, G183 of the amino acid sequence of the β5 polypeptide (mature) set forth in SEQ ID NO:7.

Provided herein is an isolated β5 polypeptide or a variant thereof having β5 activity (chymotrypsin-like) comprising one or more substitutions. In some embodiments, the β5 polypeptide comprises 1, 2, 3, 4, 5, 6, 7, 8, 9, 10, 15, 20, 25, 30 or more substitutions. In some embodiments, the β5 polypeptide comprises one substitution. In some embodiments, the substitutions occur at the amino acid residues A22, V31, S130, Y169, and optionally R19, A20, T21, A27, M45, A49, A50, C52, C63 and/or G183 of the amino acid sequence set forth in SEQ ID NO: 9.

In some embodiments, the modification comprises at least one substitution of the amino acid residues A22, V31, S130, Y169, and optionally R19, A20, T21, A27, M45, A49, A50, C52, C63, and/or G183 compared to a control wild type β5 polypeptide sequence set forth in SEQ ID NO: 7.

**"Mutation of the PSMB5 gene"** refers to a mutation in the nucleic acid sequence of the PSMB5 gene resulting in a modification of the amino acid sequence of the β5 polypeptide. In some embodiments, the PSMB5 gene comprises 1, 2, 3, 4, 5, 6, 7, 8, 9, 10, 15, 20, 25, 30 or more mutations. In some embodiments, the PSMB5 gene comprises one mutation. In some embodiments, the mutation occur at one or more of the nucleotides C241, C268, A269, A565, A682, and optionally C233, C235, G236, T238, G239, C256, T310, C312, C322, G323, G326, C332, C365, A366, and/or C725 of the PSMB5 sequence as set forth in SEQ ID NO. 1.

In some embodiments, the modification of the β5 polypeptide sequence is a substitution of arginine to an amino acid selected from among alanine, leucine, isoleucine, valine, glycine, methionine, cysteine, serine, threonine, phenylalanine, tryptophan, lysine, histidine, proline, tyrosine, arginine, asparagine, glutamine, aspartic acid and glutamic acid of the amino acid residue at position 19 of the mature β5 polypeptide (SEQ ID No.7). In some embodiments, the modification is a substitution of arginine to methionine of the amino acid residue at position 19 of the mature β5 polypeptide. Slightly reworded, in some embodiments the substitution is A20T. Slightly reworded, in some embodiments the substitution is R19M.

In some embodiments, the mutation of the PSMB5 gene is a substitution of cytosine to a nucleotide selected from thymine, guanine, adenine, of the nucleotide at position 233 of the PSMB5 gene. In some embodiments, the mutation of the PSMB5 gene is a substitution of cytosine to adenine of the nucleotide at position 233 of the PSMB5 gene (SEQ ID No. 1). Slightly reworded, in some embodiments the mutation is C233A.

In some embodiments, the modification of the β5 polypeptide sequence is a substitution of alanine to an amino acid selected from among leucine, isoleucine, valine, glycine, methionine, cysteine, serine, threonine, phenylalanine, tryptophan, lysine, histidine, proline, tyrosine, arginine, asparagine, glutamine, aspartic acid and glutamic acid of the amino acid residue at position 20 of the mature β5 polypeptide (SEQ ID No.7). In some embodiments, the modification is a substitution of alanine to threonine of the amino acid residue at position 20 of the mature β5 polypeptide. Slightly reworded, in some embodiments the substitution is A20T. In some embodiments, the modification is a substitution of alanine to valine of the amino acid residue at position 20 of the mature β5 polypeptide. Slightly reworded, in some embodiments the substitution is A20V.

In some embodiments, the mutation of the PSMB5 gene is a substitution of cytosine to a nucleotide selected from thymine, guanine, adenine, of the nucleotide at position 235 of the PSMB5 gene. In some embodiments, the mutation of the PSMB5 gene is a substitution of cytosine to thymine of the nucleotide at position 235 of the PSMB5 gene (SEQ ID No. 1). Slightly reworded, in some embodiments the mutation is C235T. In some embodiments, the mutation of the PSMB5 gene is a substitution of guanine to a nucleotide selected from thymine, guanine, adenine, of the nucleotide at position 236 of the PSMB5 gene. In some embodiments, the mutation of the PSMB5 gene is a substitution of guanine to adenine of the nucleotide at position 236 of the PSMB5 gene. Slightly reworded, in some embodiments the mutation is G236A.

In some embodiments, the modification of the β5 polypeptide sequence is a substitution of threonine to an amino acid selected from among leucine, isoleucine, valine, alanine, glycine, methionine, cysteine, serine, phenylalanine, tryptophan, lysine, histidine, proline, tyrosine, arginine, asparagine, glutamine, aspartic acid and glutamic acid of the amino acid residue at position 21 of the mature β5 polypeptide (SEQ ID No.7). In other embodiments, the modification is a substitution of threonine to alanine of the amino acid residue at position 21 of the β5 polypeptide. Slightly reworded, in other embodiments the substitution is T21A. In some embodiments, the modification is a substitution of threonine to isoleucine of the amino acid residue at position 21 of the mature β5 polypeptide. Slightly reworded, in some embodiments the substitution is T20I.

In some embodiments, the mutation of the PSMB5 gene is a substitution of thymine to a nucleotide selected from cytosine, guanine, adenine, of the nucleotide at position 238 of the PSMB5 gene. In some embodiments, the mutation of the PSMB5 gene is a substitution of thymine to cytosine of the nucleotide at position 238 of the PSMB5 gene (SEQ ID No. 1). Slightly reworded, in some embodiments the mutation is T238C. In other embodiments, the mutation of the PSMB5 gene is a substitution of guanine to a nucleotide selected from thymine, cytosine, adenine, of the nucleotide at position 239 of the PSMB5 gene. In other embodiments, the mutation of the PSMB5 gene is a substitution of guanine to adenine of the nucleotide at position 239 of the PSMB5 gene. Slightly reworded, in other embodiments the mutation is G239A.

In some embodiments, the modification of the β5 polypeptide sequence is a substitution of alanine to an amino acid selected from among leucine, isoleucine, valine, threonine, glycine, methionine, cysteine, serine, phenylalanine, tryptophan, lysine, histidine, proline, tyrosine, arginine, asparagine, glutamine, aspartic acid and glutamic acid of the amino acid residue at position 22 of the mature β5 polypeptide (SEQ ID No.7). In other embodiments, the modification is a substitution of alanine to threonine of the amino acid residue at position 22 of the mature β5 polypeptide. Slightly reworded, in other embodiments the substitution is A22T.

In some embodiments, the mutation of the PSMB5 gene is a substitution of cytosine to a nucleotide selected from thymine, guanine, adenine, of the nucleotide at position 241 of the PSMB5 gene. In some embodiments, the mutation of the PSMB5 gene is a substitution of cytosine to thymine of the nucleotide at position 241 of the PSMB5 gene (SEQ ID No. 1). Slightly reworded, in some embodiments the mutation is C241T.

In some embodiments, the modification of the β5 polypeptide sequence is a substitution of alanine to an amino acid selected from among asparagine, aspartic acid, cysteine, glutamine, glutamic acid, glycine, histidine, isoleucine, leucine, lysine, methionine, phenylalanine, proline, serine, threonine, tryptophan, tyrosine, arginine, valine of the amino acid residue at position 27 of the mature β5 polypeptide (SEQ ID No.7). In other embodiments, the modification is a substitution of alanine to threonine of the amino acid residue at position 27 of the mature β5 polypeptide. Slightly reworded, in other embodiments the substitution is A27T.

In some embodiments, the mutation of the PSMB5 gene is a substitution of cytosine to a nucleotide selected from thymine, guanine, adenine, of the nucleotide at position 256 of the PSMB5 gene. In some embodiments, the mutation of the PSMB5 gene is a substitution of cytosine to thymine of the nucleotide at position 256 of the PSMB5 gene (SEQ ID No. 1). Slightly reworded, in some embodiments the mutation is C256T.

In some embodiments, the modification of the β5 polypeptide sequence is a substitution of valine to an amino acid selected from among alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine of the amino acid residue at position 31 of the mature β5 polypeptide (SEQ ID No.7). In other embodiments, the modification is a substitution of valine to leucine of the amino acid residue at position 31 of the mature β5 polypeptide. Slightly reworded, in other embodiments the substitution is V31L. In other embodiments, the modification is a substitution of valine to glutamic acid of the amino acid residue at position 31 of the mature β5 polypeptide. Slightly reworded, in other embodiments the substitution is V31E. In still other embodiments, the modification is a substitution of valine to glycine of the amino acid residue at position 31 of the mature β5 polypeptide. Slightly reworded, in still other embodiments the substitution is V31G.

In some embodiments, the mutation of the PSMB5 gene is a substitution of cytosine to a nucleotide selected from thymine, guanine, adenine, of the nucleotide at position 268 of the PSMB5 gene. In some embodiments, the mutation of the PSMB5 gene is a substitution of cytosine to adenine of the nucleotide at position 268 of the PSMB5 gene (SEQ ID No. 1). Slightly reworded, in some embodiments the substitution is C268A. In other embodiments, the mutation of the PSMB5 gene is a substitution of adenine to a nucleotide selected from thymine, guanine, cytosine, of the nucleotide at position 269 of the PSMB5 gene. In still other embodiments, the mutation of the PSMB5 gene is a substitution of adenine to thymine of the nucleotide at position 269 of the PSMB5 gene (SEQ ID No. 1). Slightly reworded, in still other embodiments the mutation is A269T. In other embodiments, the mutation of the PSMB5 gene is a substitution of adenine to cytosine of the nucleotide at position 269 of the PSMB5 gene (SEQ ID No. 1). Slightly reworded, in still other embodiments the mutation is A269C.

In some embodiments, the modification of the β5 polypeptide sequence is a substitution of methionine to an amino acid selected from among alanine, arginine, asparagine, aspartic acid, cysteine, glutamine, glutamic acid, glycine, histidine, isoleucine, leucine, lysine, phenylalanine, proline, serine, threonine, tryptophan, tyrosine, valine of the amino acid residue at position 45 of the mature β5 polypeptide (SEQ ID No.7). In other embodiments, the modification is a substitution of methionine to valine of the amino acid residue at position 45 of the β5 polypeptide. Slightly reworded, in other embodiments the substitution is M45V. In other embodiments, the modification is a substitution of methionine to isoleucine of the amino acid residue at position 45 of the β5 polypeptide. Slightly reworded, in other embodiments the substitution is M45I.

In some embodiments, the mutation of the PSMB5 gene is a substitution of thymine to a nucleotide selected from cytosine, guanine, and adenine of the nucleotide at position 310 of the PSMB5 gene. In some embodiments, the mutation of the PSMB5 gene is a substitution of thymine to cytosine of the nucleotide at position 310 of the PSMB5 gene (SEQ ID No. 1). Slightly reworded, in some embodiments the mutation is T310C. In other embodiments, the mutation of the PSMB5 gene is a substitution of cytosine to a nucleotide selected from thymine, guanine, adenine, of the nucleotide at position 312 of the PSMB5 gene. In still other embodiments, the mutation of the PSMB5 gene is a substitution of cytosine to adenine of the nucleotide at position 312 of the PSMB5 gene (SEQ ID No. 1). Slightly reworded, in still other embodiments the mutation is C312A.

In some embodiments, the modification of the β5 polypeptide sequence is a substitution of alanine to an amino acid selected from among arginine, asparagine, aspartic acid, cysteine, glutamine, glutamic acid, glycine, histidine, isoleucine, leucine, lysine, methionine phenylalanine, proline, serine, threonine, tryptophan, tyrosine, valine of the amino acid residue at position 49 of the mature β5 polypeptide (SEQ ID No.7). In other embodiments, the modification is a substitution of alanine to serine of the amino acid residue at position 49 of the β5 polypeptide. Slightly reworded, in other embodiments the substitution is A49S. In still other embodiments, the modification is a substitution of alanine to threonine of the amino acid residue at position 49 of the β5 polypeptide. Slightly reworded, in still other embodiments the substitution is A49T. In still some other embodiments, the modification is a substitution of alanine to glutamic acid of the amino acid residue at position 49 of the β5 polypeptide. Slightly reworded, in still some other embodiments the substitution is A49E.

In some embodiments, the mutation of the PSMB5 gene is a substitution of cytosine to a nucleotide selected from thymine, guanine, and adenine of the nucleotide at position 322 of the PSMB5 gene. In some embodiments, the mutation of the PSMB5 gene is a substitution of cytosine to alanine of the nucleotide at position 322 of the PSMB5 gene (SEQ ID No. 1). Slightly reworded, in some embodiments the substitution is C322A. In other embodiments, the mutation of the PSMB5 gene is a substitution of cytosine to a thymine of the nucleotide at position 322 of the PSMB5 gene. Slightly reworded, in other embodiments the mutation is C322T. In still other embodiments, the mutation of the PSMB5 gene is a substitution of guanine to a nucleotide selected from thymine, cytosine, and adenine of the nucleotide at position 323 of the PSMB5 gene (SEQ ID No. 1). In other embodiments, the mutation of the PSMB5 gene is a substitution of guanine to a thymine of the nucleotide at position 323 of the PSMB5 gene. Slightly reworded, in still other embodiments the mutation is C323T. In further embodiments, the mutation of the PSMB5 gene is a substitution of guanine to an adenine of the nucleotide at position 323 of the PSMB5 gene. Slightly reworded, in further embodiments the mutation is G323A.

In some embodiments, the modification of the β5 polypeptide sequence is a substitution of alanine to an amino acid selected from among arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine of the amino acid residue at position 50 of the mature β5 polypeptide (SEQ ID No.7). In other embodiments, the modification is a substitution of alanine to valine of the amino acid residue at position 50 of the β5 polypeptide. Slightly reworded, in other embodiments the substitution is A50V.

In some embodiments, the mutation of the PSMB5 gene is a substitution of guanine to a nucleotide selected from cytosine, thymine, and adenine of the nucleotide at position 326 of the PSMB5 gene. In some embodiments, the mutation of the PSMB5 gene is a substitution of guanine to adenine of the nucleotide at position 326 of the PSMB5 gene (SEQ ID No. 1). Slightly reworded, in some embodiments the mutation is G326A.

In some embodiments, the modification of the β5 polypeptide sequence is a substitution of cysteine to an amino acid selected from among alanine, arginine, asparagine, aspartic acid, glutamine, glutamic acid, glycine, histidine, isoleucine, leucine, lysine, methionine, phenylalanine, proline, serine, threonine, tryptophan, tyrosine, valine of the amino acid residue at position 52 of the mature β5 polypeptide (SEQ ID No.7). In other embodiments, the modification is a substitution of cysteine to phenylalanine of the amino acid residue at position 52 of the β5 polypeptide. Slightly reworded, in other embodiments the substitution is C52F.

In some embodiments, the mutation of the PSMB5 gene is a substitution of cytosine to a nucleotide selected from guanine, thymine, and adenine of the nucleotide at position 332 of the PSMB5 gene. In some embodiments, the mutation of the PSMB5 gene is a substitution of cytosine to adenine of the nucleotide at position 332 of the PSMB5 gene (SEQ ID No. 1). Slightly reworded, in some embodiments the mutation is C332A.

In some embodiments, the modification of the β5 polypeptide sequence is a substitution of cysteine to an amino acid selected from among alanine, arginine, asparagine, aspartic acid, glutamine, glutamic acid, glycine, histidine, isoleucine, leucine, lysine, methionine, phenylalanine, proline, serine, threonine, tryptophan, tyrosine, valine of the amino acid residue at position 63 of the mature β5 polypeptide (SEQ ID No.7). In other embodiments, the modification is a substitution of cysteine to phenylalanine of the amino acid residue at position 63 of the β5 polypeptide. Slightly reworded, in other embodiments the substitution is C63P. In still other embodiments, the modification is a substitution of cysteine to tyrosine of the amino acid residue at position 63 of the β5 polypeptide. Slightly reworded, in still other embodiments the substitution is C63Y. In further embodiments, the modification is a substitution of cysteine to tryptophan of the amino acid residue at position 63 of the β5 polypeptide. Slightly reworded, in further embodiments the substitution is C63W.

In some embodiments, the mutation of the PSMB5 gene is a substitution of cytosine to a nucleotide selected from thymine, guanine, and adenine of the nucleotide at position 365 of the PSMB5 gene. In some embodiments, the mutation of the PSMB5 gene is a substitution of cytosine to adenine of the nucleotide at position 365 of the PSMB5 gene (SEQ ID No. 1). Slightly reworded, in some embodiments the substitution is C365A. In still other embodiments, the mutation of the PSMB5 gene is a substitution of cytosine to thymine of the nucleotide at position 365 of the PSMB5 gene. Slightly reworded, in some embodiments the substitution is C365T. In further embodiments, the mutation of the PSMB5 gene is a substitution of adenine to a nucleotide selected from thymine, guanine, and cytosine of the nucleotide at position 366 of the PSMB5 gene. In other further embodiments, the mutation of the PSMB5 gene is a substitution of adenine to cytosine of the nucleotide at position 366 of the PSMB5 gene (SEQ ID No. 1). Slightly reworded, in other further embodiments the mutation is A366C.

In some embodiments, the modification of the β5 polypeptide sequence is a substitution of serine to an amino acid selected from among alanine, arginine, asparagine, aspartic acid, cysteine, glutamine, glutamic acid, glycine, histidine, isoleucine, leucine, lysine, methionine, phenylalanine, proline, threonine, tryptophan, tyrosine, valine of the amino acid residue at position 130 of the mature β5 polypeptide (SEQ ID No.7). In other embodiments, the modification is a substitution of serine to alanine of the amino acid residue at position 130 of the β5 polypeptide. Slightly reworded, in other embodiments the substitution is S130A.

In some embodiments, the mutation of the PSMB5 gene is a substitution of adenine to a nucleotide selected from cytosine, guanine, and thymine of the nucleotide at position 565 of the PSMB5 gene. In some embodiments, the mutation of the PSMB5 gene is a substitution of adenine to cytosine of the nucleotide at position 565 of the PSMB5 gene (SEQ ID No. 1). Slightly reworded, in some embodiments the substitution is A565C.

In some embodiments, the modification of the β5 polypeptide sequence is a substitution of tyrosine to an amino acid selected from among alanine, arginine, asparagine, aspartic acid, cysteine, glutamine, glutamic acid, glycine, histidine, isoleucine, leucine, lysine, methionine, phenylalanine, proline, serine, threonine, tryptophan, valine of the amino acid residue at position 169 of the mature β5 polypeptide (SEQ ID No.7). In other embodiments, the modification is a substitution of tyrosine to aspartic acid of the amino acid residue at position 169 of the β5 polypeptide. Slightly reworded, in other embodiments the substitution is Y169D. In still other embodiments, the modification is a substitution of tyrosine to histidine of the amino acid residue at position 169 of the β5 polypeptide. Slightly reworded, in still other embodiments the substitution is Y169H. In further embodiments, the modification is a substitution of tyrosine to asparagine of the amino acid residue at position 169 of the β5 polypeptide. Slightly reworded, in further embodiments the substitution is Y169N.

In some embodiments, the mutation of the PSMB5 gene is a substitution of adenine to a nucleotide selected from cytosine, guanine, and thymine of the nucleotide at position 682 of the PSMB5 gene. In some embodiments, the mutation of the PSMB5 gene is a substitution of adenine to cytosine of the nucleotide at position 682 of the PSMB5 gene (SEQ ID No. 1). Slightly reworded, in some embodiments the substitution is A682C. In other embodiments, the mutation of the PSMB5 gene is a substitution of adenine to guanine of the nucleotide at position 682 of the PSMB5 gene. Slightly reworded, in other embodiments the substitution is A682G. In still other embodiments, the mutation of the PSMB5 gene is a substitution of adenine to thymine of the nucleotide at position 682 of the PSMB5 gene (SEQ ID No. 1). Slightly reworded, in still other embodiments the substitution is A682T.

In some embodiments, the modification of the β5 polypeptide sequence is a substitution of glycine to an amino acid selected from among alanine, arginine, asparagine, aspartic acid, cysteine, glutamine, glutamic acid, histidine, isoleucine, leucine, lysine, methionine, phenylalanine, proline, serine, threonine, tryptophan, tyrosine, valine of the amino acid residue at position 183 of the mature β5 polypeptide (SEQ ID No.7). In other embodiments, the modification is a substitution of glycine to aspartic acid of the amino acid residue at position 183 of the β5 polypeptide. Slightly reworded, in other embodiments the substitution is G183D.

In some embodiments, the mutation of the PSMB5 gene is a substitution of cytosine to a nucleotide selected from guanine, thymine, and adenine of the nucleotide at position 725 of the PSMB5 gene. In some embodiments, the mutation of the PSMB5 gene is a substitution of cytosine to thymine of the nucleotide at position 725 of the PSMB5 gene (SEQ ID No. 1). Slightly reworded, in some embodiments the substitution is C725T.

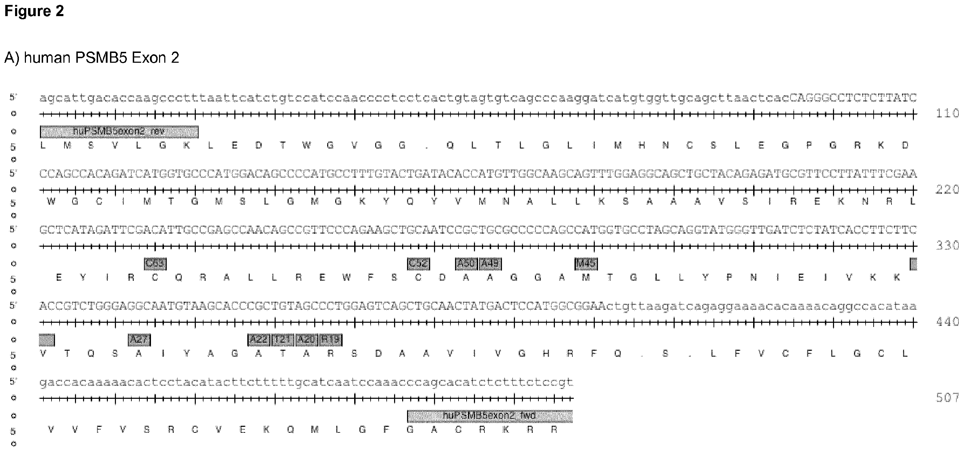

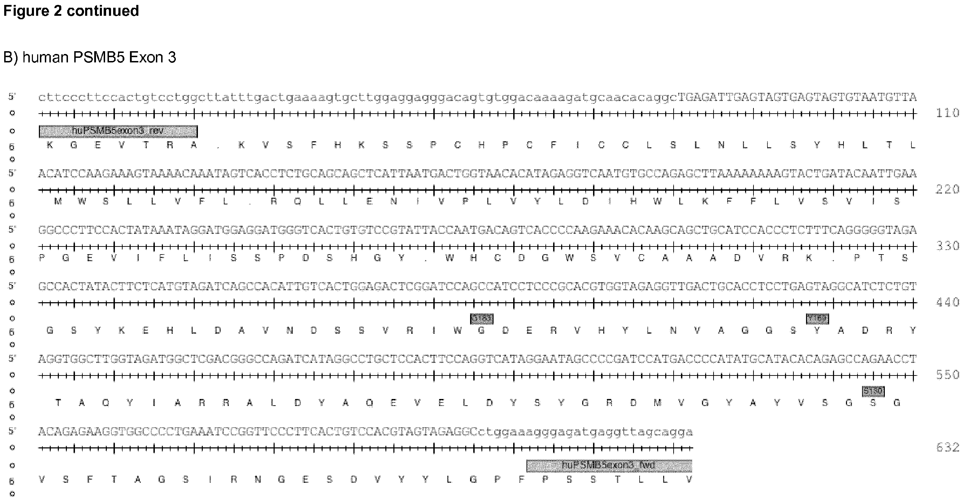

A graphical representation of the disclosed mutations in the PSMB5 gene and the corresponding substitutions in the mature β5 polypeptide is reported in Figure 2.

### Detection of mutations in the nucleic acid sequence.

Any test able to detect mutations appropriate to the type of genetic material (e.g., genomic DNA (gDNA), cDNA, RNA) may be employed. The assaying may comprise obtaining from the biological sample containing diseased cells the sequence of at least a portion of the nucleic acid sequence of PSMB5. In an embodiment, the method may further comprise a''') comparing the PSMB5 sequence in the diseased cells from the patient to the PSMB5 sequence in non-diseased control cells (e.g., the wild type genetic sequence), a'''') identifying any difference between the PSMB5 sequence in the diseased cells from the patient and in the non-diseased control cells, in order to detect a mutation at one or more of the nucleotides C241, C268, A269, A565, A682, and optionally C233, C235, G236, T238, G239, C256, T310, C312, C322, G323, G326, C332, C365, A366, and/or C725 of the PSMB5 sequence as set forth in SEQ ID NO. 1. Slightly reworded, the method may further comprise a''') comparing the PSMB5 sequence in the diseased cells from the patient to the PSMB5 sequence in non-diseased control cells (e.g., the wild type genetic sequence), a'''') identifying any difference between the PSMB5 sequence in the diseased cells from the patient and in the non-diseased control cells, in order to detect a mutation at one or more of the nucleotides that results in a substitution in at least one of the amino acid residues A22, V31, S130, Y169, and optionally R19, A20, T21, A27, M45, A49, A50, C52, C63 and/or G183 of the amino acid sequence of the β5 polypeptide (mature) set forth in SEQ ID NO.7. Thus, the method according to the invention may comprise sequencing a nucleic acid such as DNA or RNA of the PSMB5 in diseased cells from the patient and optionally in non-diseased control cells.

As used herein, the term **"control"** refers to a suitable non-hematological malignancy cell, including, for example cells from a subject or a group of subjects who are healthy, and especially do not have a hematological or leukemic disorder.

The genetic material (such as DNA or RNA) may be obtained directly from a biological sample containing diseased cells of the patient, or can be copied or amplified from the genetic material within the biological sample containing diseased cells (e.g., via polymerase chain reaction (PCR), reverse transcription polymerase chain reaction (RT-PCR), or other suitable technique). To ensure that a sufficient quantity of genetic material is available for testing, the genetic material may be amplified from the diseased cells obtained from the patient, and the amplified genetic material is assayed in accordance with the inventive method. Preferably, a PCR or RT-PCR strategy is employed using primers flanking all or a portion of PSMB5 as set forth in the sequences SEQ ID No.3-6, and depicted on Figure 2, so as to amplify this sequence from the biological sample containing diseased cells or from the diseased cells of the patient under analysis.

The term **"primer"** as used herein refers to a DNA oligonucleotide, either single-stranded or double-stranded, either derived from a biological system, generated by restriction enzyme digestion, or produced synthetically which, when placed in the proper environment, is able to functionally act as an initiator of template-dependent nucleic acid synthesis. When presented with an appropriate nucleic acid template, suitable nucleoside triphosphate precursors of nucleic acids, a polymerase enzyme, suitable cofactors and conditions such as a suitable temperature and pH, the primer may be extended at its 3' terminus by the addition of nucleotides by the action of a polymerase or similar activity to yield a primer extension product. The primer may vary in length depending on the particular conditions and requirement of the application. For example, in diagnostic applications, the oligonucleotide primer is typically 15-25 or more nucleotides in length. The primer must be of sufficient complementarity to the desired template to prime the synthesis of the desired extension product, that is, to be able anneal with the desired template strand in a manner sufficient to provide the 3' hydroxyl moiety of the primer in appropriate juxtaposition for use in the initiation of synthesis by a polymerase or similar enzyme. It is not required that the primer sequence represent an exact complement of the desired template. For example, a non-complementary nucleotide sequence may be attached to the 5' end of an otherwise complementary primer. Alternatively, non-complementary bases may be interspersed within the oligonucleotide primer sequence, provided that the primer sequence has sufficient complementarity with the sequence of the desired template strand to functionally provide a template-primer complex for the synthesis of the extension product.

**"Polymerase chain reaction"** is abbreviated PCR and means an *in vitro* method for enzymatically amplifying specific nucleic acid sequences. PCR involves a repetitive series of temperature cycles with each cycle comprising three stages: denaturation of the template nucleic acid to separate the strands of the target molecule, annealing a single stranded PCR oligonucleotide primer to the template nucleic acid, and extension of the annealed primer(s) by DNA polymerase. PCR provides a means to detect the presence of the target molecule and, under quantitative or semiquantitative conditions, to determine the relative amount of that target molecule within the starting pool of nucleic acids.

The term **"nucleic acid"** refers to deoxyribonucleotides, deoxyribonucleosides, ribonucleosides, or ribonucleotides and polymers thereof in either single- or double-stranded form. Unless specifically limited, the term encompasses nucleic acids containing known analogs of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (including but not limited to, degenerate codon substitutions) and complementary sequences as well as the sequence explicitly indicated.
It is preferred for all *in vitro* methods disclosed herein that determining the nucleic acid sequence comprises DNA sequencing.

Consequently, the present application is directed to an *in vitro* method for identifying resistance to bortezomib treatment in a patient prior, during or after bortezomib treatment comprising the steps:
a) obtaining a biological sample containing diseased cells from said patient,
   a') determining the nucleic acid sequence of the PSMB5 gene encoding the polypeptide β5 by_DNA sequencing, and
   a") detecting a mutation in the nucleic acid sequence of the PSMB5 gene that results in a substitution in the amino acid sequence of the polypeptide β5 in at least one of the amino acid residues A22, V31, S130 and Y169, and
b) detecting or thereby detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

In another preferred embodiment, the present application is directed to an *in vitro* method for identifying resistance to bortezomib treatment in a patient prior, during or after bortezomib treatment comprising the steps:
a) obtaining a biological sample containing diseased cells from said patient, and
   a') determining the nucleic acid sequence of the PSMB5 gene encoding the polypeptide β5 by_DNA sequencing, and
   a") detecting a mutation in the nucleic acid sequence of the PSMB5 gene that results in a substitution in the amino acid sequence of the polypeptide β5 in at least one of the amino acid residues A22, V31, S130, and Y169, and detecting a mutation in the nucleic acid sequence of the PSMB5 gene that results in a substitution in the amino acid sequence of the polypeptide β5 in at least one of the amino acid residues R19, A20, T21, A27, M45, A49, A50, C52, C63, and G183,
b) detecting or thereby detecting a substitution in the amino acid sequence of the polypeptide β5 (PSMB5) in at least one of the amino acid residues A22, V31, S130, and Y169, and optionally detecting or thereby detecting a substitution in the amino acid sequence of the polypeptide β5 (PSMB5) in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.

### Composition or synergistic composition of bortezomib and a further drug

It was found that the a patient with an identified bortezomib resistance and suffering from a proteasome inhibitor sensitive diseases, especially multiple myeloma, mantle cell lymphoma, non-Hodgkin's lymphoma, TEMPI syndrome, light chain deposition disease (LCDD), IgG₄-related disease, and scleromyxedema can still successfully be treated with a certain other drug instead of bortezomib that is not effected by a substitution in the β5 polypeptide sequence of the amino acid residues A22, V31, S130 or Y169 .

Therefore an especially preferred embodiment of the present invention relates to a composition or synergistic composition of bortezomib and at least one further drug suitable for the treatment of a disease selected from the group comprising multiple myeloma, mantle cell lymphoma, non-Hodgkin's lymphoma, TEMPI syndrome, light chain deposition disease (LCDD), IgG₄-related disease, or scleromyxedema, wherein a substitution in the amino acid sequence of the polypeptide β5 in at least one of the amino acid residues A22, V31, S130 and Y169 does not result in resistance to that further drug.

A further especially preferred embodiment of the present invention relates to a composition or synergistic composition of bortezomib and at least one further drug suitable for the treatment of a disease selected from the group comprising multiple myeloma, mantle cell lymphoma, non-Hodgkin's lymphoma, TEMPI syndrome, light chain deposition disease (LCDD), IgG₄-related disease, or scleromyxedema, wherein a substitution in the amino acid sequence of the mature polypeptide β5 in at least one of the amino acid residues R19, A20, T21, A27, M45, A49, A50, C52, C63, and G183 of mature polypeptide β5 does not result in resistance to that further drug.

"Synergistic" or "synergy" is used herein to mean that the effect is more than its additive property. In preferred embodiments, the synergy is at least 1.5, 2, 5, or 10 fold.

A **"composition"** (also referred to as "combination") means more than one item, e.g. a compound such as a second generation proteasome inhibitor, a therapeutic antibody, or an immunomodulatory drug.
The present disclosure also relates to composition, pharmaceuticals, and pharmaceutical compositions containing the described composition. The two components of the synergistic composition of the present invention, e.g. the bortezomib and the further suitable drug, may be administered together, or separately. When administered together, the two components may be formulated together in one pharmaceutical composition, which may include a pharmaceutical acceptable carrier or excipient.
Alternatively, the two components might also be formulated in different pharmaceutical compositions. In this case the two components can be administered simultaneously or subsequently. In an embodiment, the bortezomib is administered prior to and/or separately from the administration of the other drug. In embodiments, the composition is administered in an effective amount.

The term **"suitable drug"** comprises second generation proteasome inhibitors, immunomodulatory drugs, and recently developed therapeutic monoclonal antibodies.

Immunomodulatory drugs are a group of compounds that are analogues of thalidomide, a glutamic acid derivative with anti-angiogenic properties and potent anti-inflammatory effects owing to its anti-tumour necrosis factor alpha (TNFα) activity. Thalidomide analogues were initially synthesized with the aim of optimizing both anti-TNFa and anti-angiogenic properties while reducing toxicities. The two leading immunomodulatory drugs , lenalidomide (CC-5013; IMiD3; Revlimid) and pomalidomide (CC-4047; IMiD1; Acti-mid) were the first to enter clinical trials in multiple myeloma in 1999, and are now the subject of clinical evaluation in other haematological malignancies. The precise cellular targets and the exact mechanism of actions of immunomodulatory drugs in multiple myeloma remains unclear, however preclinical studies have unveiled multiple effects including anti-proliferative, T-cell co-stimulatory, anti-angiogenic and anti-inflammatory effects.
Thalomid® (thalidomide) in combination with dexamethasone is indicated for the treatment of patients with newly diagnosed multiple myeloma, and is marketed by Celgene. The "immunomodulatory drugs" used herein comprise Thalomid® (thalidomide), Lenalidomide, Pomalidomide,

Lenalidomide (CC-5013, Revlimid™) and Pomalidomide (CC4047, Actimid™) are "thalidomide analogs". The term refers to a synthetic chemical compound using the thalidomide structure as a backbone (e.g., side groups have been added or such groups have been deleted from the parent structure). The analog differs in structure from thalidomide and its metabolite compounds such as by a difference in the length of an alkyl chain, a molecular fragment, by one or more functional groups, or a change in ionization. The term "thalidomide analog" also includes the metabolites of thalidomide. Thalidomide analogs include the racemic mixture of the S- and the R-enantiomer of a respective compound and the S-enantiomer or to the R-enantiomer individually. The racemic mixture is preferred.

In the context of the present invention, the term **"antibody"** covers monoclonal antibodies, polyclonal antibodies, multispecific antibodies (e.g. bispecific antibodies) formed from at least two antibodies, antibody fragments and derivatives thereof if they exhibit the desired activity. The antibody may be an IgM, IgG, e.g. IgG₁, IgG₂, IgG₃ or IgG₄. Antibody fragments comprise a portion of an antibody, generally the antigen binding or variable region of the intact antibody. Examples of antibody fragments include Fab, Fab', F (ab') 2 and Fv fragments, diabodies, single chain antibody molecules and multispecific antibody fragments. A bispecific monoclonal antibody (BsMAb, BsAb) is an artificial protein made from two different monoclonal antibodies that can bind to two different types of antigen simultaneously. Particularly, the antibody may be a recombinant antibody or antibody fragment, especially selected from chimeric antibodies or fragments thereof and diabodies. For therapeutic purposes, particularly for the treatment of humans, the administration of chimeric antibodies, humanized antibodies or human antibodies is especially preferred.

Examples of recently developed antibodies are anti-SLAM7 Elotuzumab, Mapatumumab, Siltuximabb. It has been shown that bortezomib enhances activity of monoclonal antibodies by rendering MM cells more vulnerable to NK cell - mediated antibody-dependent cell-mediated cytotoxicity (ADCC).

A **"proteasome inhibitor"** refers to a compound that blocks the action of proteasomes. Several classes of proteasome inhibitors are known. The class of the peptide boronates includes bortezomib (INN, PS-341; Velcade®). Another peptide boronate is delanzomib (CEP-18770). Other classes of proteasome inhibitors include peptide aldehydes (e.g. MG132), peptide vinyl sulfones, peptide epoxyketones (e.g. epoxomicin, carfilzomib), β lactone inhibitors (e.g. lactacystin, MLN 519, NPI-0052, Salinosporamide A), compounds which create dithiocarbamate complexes with metals (e.g. disulfiram, a drug which is also used for the treatment of chronic alcoholism), and certain antioxidants (e.g. epigallocatechin-3-gallate) catechin-3-gallate, and salinosporamide A.

While ixazomib, as bortezomib, is a boronic acid and a reversible PSMB5 inhibitor, the epoxyketones carfilzomib and oprozomib (investigational agent) inhibit irreversibly.
As disclosed herein, preferred **"second generation proteasome inhibitors"** comprise carfilzomib (PR-171), oprozomib (ONX-0912), ixazomib (MLN9708 / MLN2238), delanzomib (CEP-18770), and marizomib (NPI-0052), syringolins, Syringolin analogues.

Therefore, a further aspect of the present invention is directed to a composition or a synergistic composition of bortezomib and at least one further drug suitable for the treatment of a disease selected from the group comprising or consisting of multiple myeloma, mantle cell lymphoma, non-Hodgkin's lymphoma, TEMPI syndrome, light chain deposition disease (LCDD), IgG₄-related disease, or scleromyxedema, wherein a substitution in the amino acid sequence of the polypeptide β5 in at least one of the amino acid residues A22, V31, S130 and Y169 or optionally in the amino acid residues R19, A20, T21, A27, M45, A49, A50, C52, C63, and G183 does not result in resistance to that further drug.

Preferably this further drug is a second generation proteasome inhibitor selected from the group consisting of carfilzomib (PR-171), oprozomib (ONX-0912), ixazomib (MLN9708 / MLN2238), delanzomib (CEP-18770), and marizomib (NPI-0052), syringolins, syringolin analogues.

Also preferred is that said further drug is an immunomodulatory drug selected from the group consisting of lenalidomide, pomalidomide, and thalidomide ....

Further preferred is that said further drug is a monoclonal antibody selected from the group consisting of anti-IL6, anti CXC4, cd38, anti SLAM7 (elotuzumab)....

### Kit for identifying resistance to bortezomib treatment.

The present invention also provides a kit for the identification of a resistance to bortezomib, which involves the detection of one or more mutations in the PSMB5 gene, encoding the β5 polypeptide, that result in one or more substitutions in the amino acid sequence of the polypeptide β5 in at least one of the amino acid residues A22, V31, S130, and Y169, and optionally in the amino acid residues R19, A20, T21, A27, M45, A49, A50, C52, C63 and G183, wherein the detected substitution inhibits the binding of bortezomib to the active site of the polypeptide β5, thereby identifying resistance to bortezomib treatment.

Thus, an embodiment of the present invention refers to a diagnostic kit for identifying resistance to bortezomib treatment in a patient suffering from a proteasome inhibitor sensitive disease comprising:
i) an extraction system comprising materials to isolate RNA or gDNA, and
ii) sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues A22, V31, S130 and Y169, and
iii) means for revealing the polymerase chain reaction amplification of step (ii).

A further preferred diagnostic kit for identifying resistance to bortezomib treatment in a patient suffering from a proteasome inhibitor sensitive disease comprises:
i) an extraction system comprising materials to isolate RNA or gDNA, and
ii) sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues A22, V31, S130 and Y169, and further sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues R19, A20, T21, A27, M45, A49, A50, C52, C63, and G183
iii) means for revealing the polymerase chain reaction amplification of step (ii).

Slightly reworded, an embodiment of the present invention refers to a diagnostic kit for identifying resistance to bortezomib treatment in a patient in need thereof:
i) an extraction system comprising materials to isolate RNA or gDNA, and
ii) sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues A22, V31, S130 and Y169, and
iii) means for revealing the polymerase chain reaction amplification of step (ii).

Thus, a further preferred diagnostic kit for identifying resistance to bortezomib treatment in a patient in need thereof:
i) an extraction system comprising materials to isolate RNA or gDNA, and
ii) sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues A22, V31, S130 and Y169, and further sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues R19, A20, T21, A27, M45, A49, A50, C52, C63, and G183
iii) means for revealing the polymerase chain reaction amplification of step (ii).

A particularly preferred embodiment refers to the use of the diagnostic kit comprising:
i) an extraction system comprising materials to isolate RNA or gDNA, and
ii) sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues A22, V31, S130 and Y169, and
iii) means for revealing the polymerase chain reaction amplification of step (ii),
for identifying resistance to bortezomib treatment in a patient having a substitution in the amino acid sequence of the polypeptide β5 in at least one of the amino acid residues A22, V31, S130 and Y169.

Also preferred is the use of the diagnostic kit comprising:
i) an extraction system comprising materials to isolate RNA or gDNA, and
ii) sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues A22, V31, S130 and Y169, and further sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues R19, A20, T21, A27, M45, A49, A50, C52, C63, and G183
iii) means for revealing the polymerase chain reaction amplification of step (ii),
for identifying resistance to bortezomib treatment in a patient having a substitution in the amino acid sequence of the polypeptide β5 in at least one of the amino acid residues A22, V31, S130 and Y169 and optionally in at least one of the amino acid residues R19, A20, T21, A27, M45, A49, A50, C52, C63, and G183.

The kits as disclosed herein are also very useful for providing an alternative treatment for the patient with a resistance to bortezomib treatment.

### Uses of the disclosed method

In an aspect the method to identify a bortezomib resistance in a patient is used for the treatment of a proteasome inhibitor sensitive disease selected from the group comprising multiple myeloma and/or non-hodgkins lymphoma, mantle cell lymphoma, TEMPI syndrome, light chain deposition disease (LCDD), IgG₄-related disease, or scleromyxedema.

Therefore, according to a preferred embodiment, the present invention further provides a method for the treatment of a patient suffering from a proteasome inhibitor sensitive disease comprising the steps of:
a) determining whether the patient is resistant to bortezomib treatment by:
   i) obtaining a biological sample containing diseased cells from said patient;
   ii) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.
b) if the patient is resistant to bortezomib treatment, then administering to the patient a therapeutically effective amount of a drug other than bortezomib and against which the detected substitution is known to not cause resistance,
c) if the patient is not resistant to bortezomib treatment, then administering to the patient a therapeutically effective amount bortezomib.

Moreover, according to another preferred embodiment, the present invention further provides a method for the treatment of a patient suffering from a proteasome inhibitor sensitive disease comprising the steps of:
a) determining whether the patient is resistant to bortezomib treatment by:
   i) obtaining a biological sample containing diseased cells from said patient;
   ii) detecting a substitution in the amino acid sequence of the polypeptide β5 (PSMB5) in at least one of the amino acid residues A22, V31, S130 and Y169, and optionally detecting a substitution in the amino acid sequence of the polypeptide β5 in at least one of the amino acid residues R19, A20, T21, A27, M45, A49, A50, C52, C63, and G183, wherein said substitution inhibits the binding of bortezomib to the active site of the polypeptide β5, thereby identifying said patient as being resistant to bortezomib treatment.
b) if the patient is resistant to bortezomib treatment, then administering to the patient a therapeutically effective amount of a drug other than bortezomib and against which the detected substitution is known to not cause resistance,
c) if the patient is not resistant to bortezomib treatment, then administering to the patient a therapeutically effective amount bortezomib.

**"Administered"** or **"administration"** includes but is not limited to delivery by an injectable form, such as, for example, an intravenous, intramuscular, intradermal or subcutaneous route or mucosal route, for example, as a nasal spray or aerosol for inhalation or as an ingestable solution, capsule or tablet.

A **"therapeutically effective amount"** of a compound or composition refers to an amount sufficient to cure, alleviate or partially arrest the clinical manifestations of a given disease or disorder and its complications. The amount that is effective for a particular therapeutic purpose will depend on the severity of the disease or injury as well as on the weight and general state of the subject. It will be understood that determination of an appropriate dosage may be achieved, using routine experimentation, by constructing a matrix of values and testing different points in the matrix, all of which is within the ordinary skills of a trained physician or clinical scientist.

The term **"treating"** or **"treatment"** as used herein and as is well understood in the art, means an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, diminishment of the reoccurrence of disease, and remission (whether partial or total), whether detectable or undetectable. "Treating" and "Treatment" can also mean prolonging survival as compared to expected survival if not receiving treatment. "Treating" and "treatment" as used herein also include prophylactic treatment. For example, a subject with early stage multiple myeloma can be treated to prevent progression or alternatively a subject in remission can be treated with a compound or composition described herein to prevent recurrence. Treatment methods comprise administering to a subject a therapeutically effective amount of one or more compounds described in the present disclosure and optionally consists of a single administration, or alternatively comprises a series of applications. For example, the compounds described herein may be administered at least once a week, from about one time per week to about once daily for a given treatment or the compound may be administered twice daily. The length of the treatment period depends on a variety of factors, such as the severity of the disease, the age of the patient, the concentration, the activity of the compounds described herein, and/or a composition thereof. It will also be appreciated that the effective dosage of the compound used for the treatment or prophylaxis may increase or decrease over the course of a particular treatment or prophylaxis regime. Changes in dosage may result and become apparent by standard diagnostic assays known in the art. In some instances, chronic administration may be required.

The **dosage administered** will vary depending on the use and known factors such as the pharmacodynamic characteristics of the particular substance, and its mode and route of administration, age, health, and weight of the individual recipient, nature and extent of symptoms, kind of concurrent treatment, frequency of treatment, and the effect desired. Dosage regime may be adjusted to provide the optimum therapeutic response.

Therefore, according to a more preferred embodiment, the present invention further provides a method for treatment of a patient suffering from a disease selected from the group comprising multiple myeloma, mantle cell lymphoma, non-Hodgkin's lymphoma, TEMPI syndrome, light chain deposition disease (LCDD), IgG₄-related disease, or scleromyxedema, comprising the steps of:
a) determining whether the patient is resistant to bortezomib treatment by:
   i) obtaining a biological sample containing diseased cells from said patient; or
   i) obtaining a biological sample containing diseased cells from bone marrow, whole blood, mononuclear cells, plasma cells, from said patient; and
   ii) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment,
b1) if the patient is resistant to bortezomib treatment, then administering to the patient a therapeutically effective amount of a drug other than bortezomib against which the detected substitution is known to not cause resistance,
b2) if the patient is slightly resistant to bortezomib treatment, then administering to the patient a therapeutically effective amount of a composition of bortezomib and a further drug other than bortezomib against which the detected substitution is known to not cause resistance,
b3) if the patient is not resistant to bortezomib treatment, then administering to the patient a therapeutically effective amount bortezomib.

Thus, according to a more preferred embodiment, the present invention further provides a method for treatment of a patient suffering from a disease selected from the group comprising multiple myeloma, mantle cell lymphoma, non-Hodgkin's lymphoma, TEMPI syndrome, light chain deposition disease (LCDD), IgG₄-related disease, or scleromyxedema, comprising the steps of:
a) determining whether the patient is resistant to bortezomib treatment by:
   i) obtaining a biological sample containing diseased cells from said patient; or
   i) obtaining a biological sample containing diseased cells from bone marrow, whole blood, mononuclear cells, plasma cells, from said patient; and
   ii) detecting a substitution in the amino acid sequence of the polypeptide β5 (PSMB5) in at least one of the amino acid residues A22, V31, S130 and Y169 and optionally detecting a substitution in the amino acid sequence of the polypeptide β5 (PSMB5) in at least one of the amino acid residues R19, A20, A50, T21, A27, M45, A49, C52, C63, and G183, wherein said substitution inhibits the binding of bortezomib to the active site of the polypeptide β5, thereby identifying said patient as being resistant to bortezomib treatment,
b1) if the patient is resistant to bortezomib treatment, then administering to the patient a therapeutically effective amount of a drug other than bortezomib against which the detected substitution is known to not cause resistance,
b2) if the patient is slightly resistant to bortezomib treatment, then administering to the patient a therapeutically effective amount of a composition of bortezomib and a further drug other than bortezomib against which the detected substitution is known to not cause resistance,
b3) if the patient is not resistant to bortezomib treatment, then administering to the patient a therapeutically effective amount bortezomib.

In particular, according to a preferred embodiment, the present invention further provides a method for treatment of a patient suffering from a disease selected from the group comprising multiple myeloma, mantle cell lymphoma, non-Hodgkin's lymphoma, TEMPI syndrome, light chain deposition disease (LCDD), IgG4-related disease, or scleromyxedema, comprising the steps of:
a) determining whether the patient is resistant to bortezomib treatment by:
   i) obtaining a biological sample containing diseased cells from said patient; or
   i) obtaining a biological sample containing diseased cells from bone marrow, whole blood, mononuclear cells, plasma cells, from said patient; and
      a') determining the nucleic acid sequence of the PSMB5 gene encoding the polypeptide β5, and
      a") detecting a mutation in the nucleic acid sequence of the PSMB5 gene that results in a substitution in the amino acid sequence of the polypeptide β5 in at least one of the amino acid residues A22, V31, S130 and Y169, and
   ii) detecting or thereby detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment,
b1) if the patient is resistant to bortezomib treatment, then administering to the patient a therapeutically effective amount of a drug other than bortezomib against which the detected substitution is known to not cause resistance,
b2) if the patient is slightly resistant to bortezomib treatment, then administering to the patient a therapeutically effective amount of a composition of bortezomib and a further drug other than bortezomib against which the detected substitution is known to not cause resistance,
b3) if the patient is not resistant to bortezomib treatment, then administering to the patient a therapeutically effective amount bortezomib.

Finally, a preferred embodiment of the present invention also provides a method for treatment of a patient suffering from a disease selected from the group comprising multiple myeloma, mantle cell lymphoma, non-Hodgkin's lymphoma, TEMPI syndrome, light chain deposition disease (LCDD), IgG4-related disease, or scleromyxedema, comprising the steps of:
a) determining whether the patient is resistant to bortezomib treatment by:
   i) obtaining a biological sample containing diseased cells from said patient; or
   i) obtaining a biological sample containing diseased cells from bone marrow, whole blood, mononuclear cells, plasma cells, from said patient; and
      a') determining the nucleic acid sequence of the PSMB5 gene encoding the polypeptide β5, and
      a") detecting a mutation in the nucleic acid sequence of the PSMB5 gene that results in a substitution in the amino acid sequence of the polypeptide β5 in at least one of the amino acid residues A22, V31, S130 and Y169, and optionally detecting a mutation in the nucleic acid sequence of the PSMB5 gene that results in a substitution in the amino acid sequence of the polypeptide β5 in at least one of the amino acid residues R19, A20, A50, T21, A27, M45, A49, C52, C63, and G183,
   ii) detecting or thereby detecting a substitution in the amino acid sequence of the polypeptide β5 (PSMB5) in at least one of the amino acid residues A22, V31, S130 and Y169, and optionally in the amino acid residues R19, A20, A50, T21, A27, M45, A49, C52, C63, and G183, wherein said substitution inhibits the binding of bortezomib to the active site of the polypeptide β5, thereby identifying said patient as being resistant to bortezomib treatment,
b1) if the patient is resistant to bortezomib treatment, then administering to the patient a therapeutically effective amount of a drug other than bortezomib against which the detected substitution is known to not cause resistance,
b2) if the patient is slightly resistant to bortezomib treatment, then administering to the patient a therapeutically effective amount of a composition of bortezomib and a further drug other than bortezomib against which the detected substitution is known to not cause resistance,
b3) if the patient is not resistant to bortezomib treatment, then administering to the patient a therapeutically effective amount bortezomib.

### Description of the Figures

**Figure 1****:** Unbiased forward genetic screen reveals 18 bortezomib resistance substitutions in the polypeptide β5. Schematic representation of experimental workflow for bortezomib resistance screen using N-ethyl-N-nitrosourea (ENU) mutagenesis.
**Figure 2****:** Schematic representation of the human PSMB5 exon 2 and exon 3 and the coded polypeptide β5 fragments. The figures show the positions of the mutant nucleotides on exon 2 and 3 and the corresponding substitutions on the amino acid sequence of the polypeptide β5.

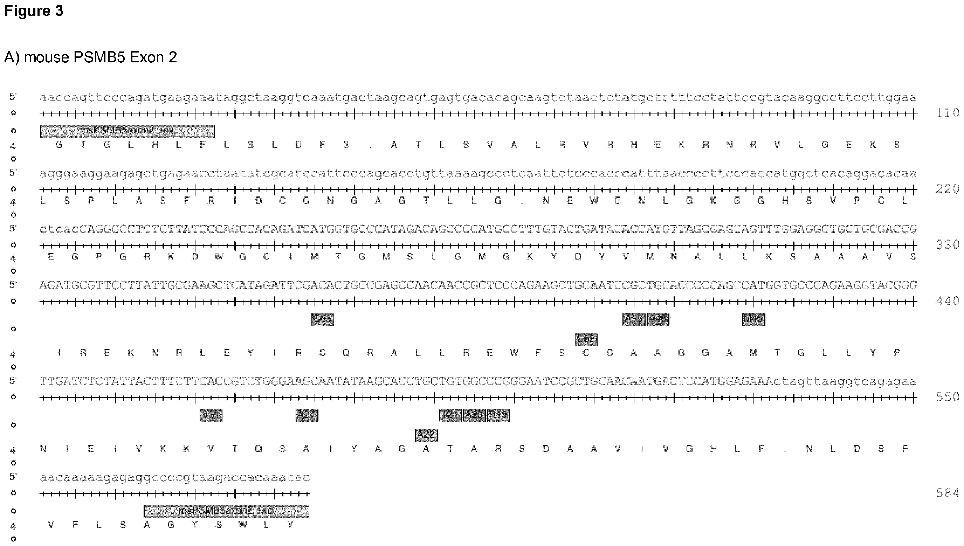

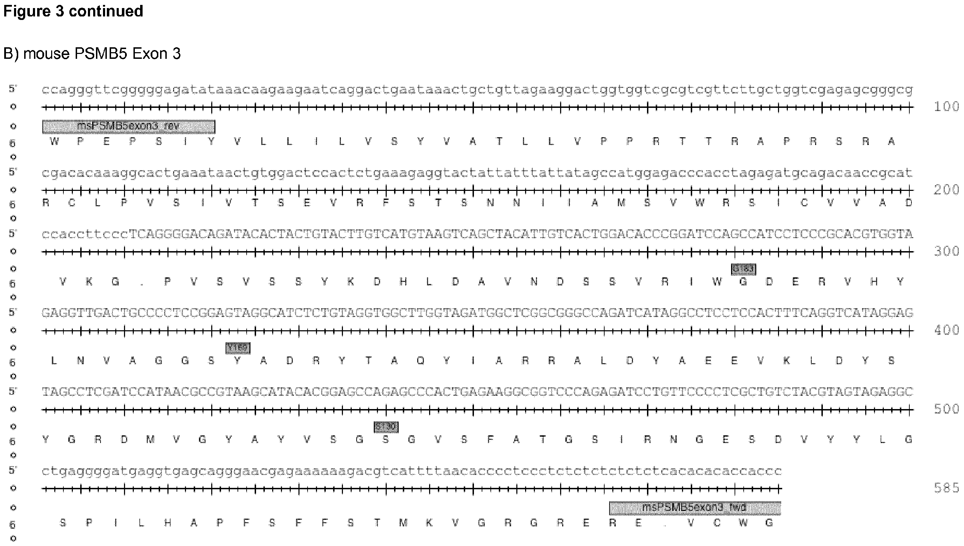

**Figure 3****:** Schematic representation of the mouse PSMB5 exon 2 and exon 3 and the coded β5 polypeptide fragments. The figures show the positions of the mutant nucleotides on exon 2 and 3 and the corresponding substitutions on the amino acid sequence of the polypeptide β5.
**Figure 4****:** Cell viability assay (XTT) of identified clones and wildtype (WT) control treated with 10 nM bortezomib. Mean ± SEM (n=4).
**Figure 5****:** Crystal structure of human polypeptide β5 (grey) in complex with bortezomib (green). Identified substitutions are highlighted in red. Hydrogen bonds between bortezomib and the amino acids in the binding pocket are shown (black dashed lines). PDB (Protein Databank): 5LF3
**Figure 6****:** (A) Crystal structure of human polypeptide β5 (grey) in complex with ixazomib (yellow). PDB: 5LF7. (B) Close-up of the binding pocket in complex with ixazomib (yellow). Identified substitutions are highlighted in red. Hydrogen bonds between proteasome inhibitors and the binding pocket are shown (black dashed lines).
**Figure 7****:** (A) Crystal structure of human polypeptide β5 (grey) in complex with carfilzomib (light orange). PDB: 4R67. (B) Close-up of the binding pocket in complex with carfilzomib (light orange). Identified substitutions are highlighted in red. Hydrogen bonds between proteasome inhibitors and the binding pocket are shown (black dashed lines).
**Figure 8****:** (A) Crystal structure of human polypeptide β5 (grey) in complex with oprozomib (light pink). PDB: 5LEZ. (B) Close-up of the binding pocket in complex with oprozomib (light pink). Identified substitutions are highlighted in red. Hydrogen bonds between proteasome inhibitors and the binding pocket are shown (black dashed lines).
**Figure 9****:** Cell viability assay of identified clones and WT control treated with 50 nM ixazomib. T21A is highlighted in pink, A49V is highlighted in orange. Mean ± SEM (n=4).
**Figure 10****:** Cell viability assay of identified clones and WT control treated with 15 nM carfilzomib. T21A is highlighted in pink, A49V is highlighted in orange. Mean ± SEM (n=4).
**Figure 11****:** Cell viability assay of identified clones and WT control treated with 75 nM oprozomib. T21A is highlighted in pink, A49V is highlighted in orange. Mean ± SEM (n=4).
**Figure 12****:** Heat map of mutants according to observed resistance. (A) Mean values of cell viability assays were entered into heatmapper62 to obtain a cluster of the mutants according to their resistance against different proteasome inhibitors. Resistance is indicated by light green color. Black means comparable to wildtype (WT). Red shows clones that are sensitive to a certain drug.

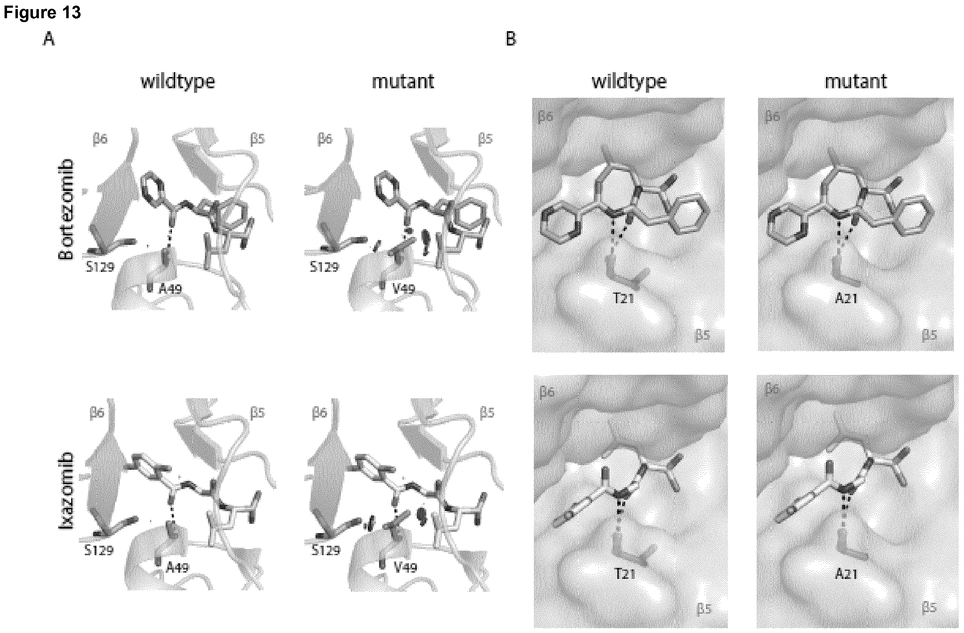

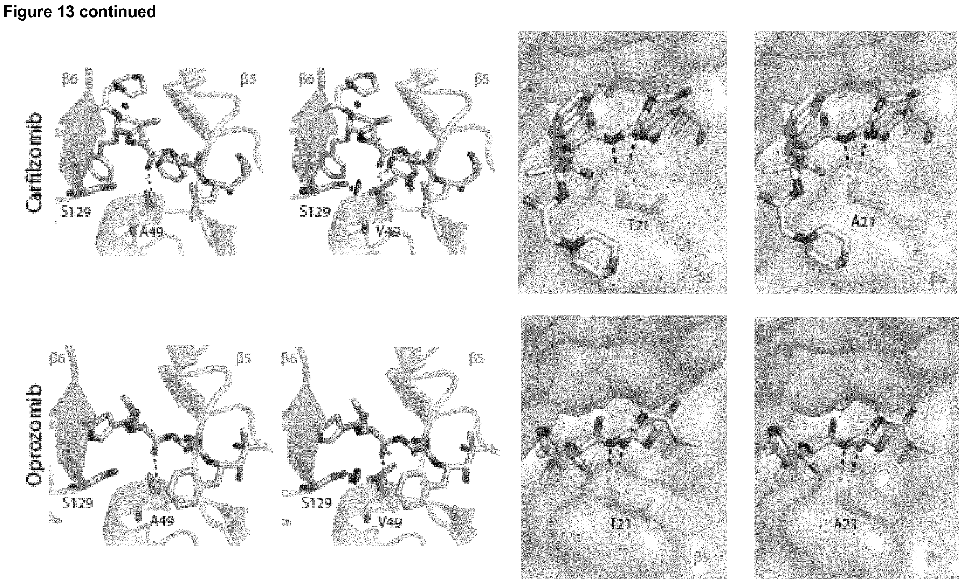

**Figure 13****:** Resistance of A49V and T21A substitutions to different proteasome inhibitors can be explained on a structural level. Crystal structure of human proteasome (β5 grey, β6 light blue) in complex with different proteasome inhibitors (bortezomib green, ixazomib yellow, carfilzomib light orange, oprozomib light pink). Hydrogen bonds are shown (black dashed lines) (A) A49 and V49 are highlighted in orange. Steric clashes of V49 with S129 of the β6 subunit and proteasome inhibitors are shown by red discs (PyMOL plugin: show_bumps). (B) T21 and A21 are highlighted in pink. PDB (Protein Data Bank): 5LF3 (bortezomib), PDB: 5LF7 (ixazomib), PDB: 4R67 (carfilzomib), PDB: 5LEZ (oprozomib). The mutagenesis tool of PyMOL was used to model the substitutions.
**Figure 14****:** Flow chart of identifying patients resistant to bortezomib or other proteasome inhibitor and choosing the appropriate therapy.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Methods:

### Cell lines and culture conditions

AN3-12 mouse haploid embryonic stem cells were cultured as previously described (Elling *et al*., 2011). In brief, the cells were grown in DMEM high glucose (Sigma-Aldrich, St. Louis, Missouri) supplemented with 15% fetal bovine serum, penicillin/streptomycin, glutamine, non-essential amino acids, sodium pyruvate (all Thermo Fisher Scientific, Waltham, Massachusetts), β-mercaptoethanol, and Leukemia-Inhibitor Factor (LIF) (both Merck Millipore, Darmstadt, Germany) on non-coated tissue culture plates.

### Cell sorting

AN3-12 cells were stained with 10 µg/ml Hoechst 33342 (Thermo Fisher Scientific, Waltham, Massachusetts) for 30 min at 37 °C. Propidium iodide (Sigma-Aldrich, St. Louis, Missouri) was used to exclude dead cells. Haploid cells were sorted using a FACSAria Fusion sorter and flow profiles were recorded with the FACSDiva software (BD, Franklin Lakes, New Jersey).

### Chemical mutagenesis

Chemical mutagenesis was performed as previously described (Horn *et al*., 2011). In brief, AN3-12 cells were transferred to 15 ml tubes, incubated with 0.1 mg/ml ENU for two hours at room temperature, washed five times with LIF-free medium, and plated on culture dishes. The cells were selected with 25 nM Bortezomib (Selleckchem, Houston, Texas) for three weeks, starting 24 h after mutagenesis. Resistant clones were transferred to 24-well plates and the Psmb5 locus was analyzed. DNA was extracted (DNA extraction solution, Epicentre, Biotechnologies, Madison, Wisconsin) and exon 2 or exon 3 of the Psmb5 gene were specifically amplified by PCR using the primers msPSMB5exon2_fwd (GTATTTGTGGTCTTACGGGGC), msPSMB5exon2_rev (AACCAGTTCCCAGATGAAGAAA), msPSMB5exon3_fwd (GGGTGGTGTGTGTGAGAGAG), and msPSMB5exon3_rev (CCAGGGTTCGGGGGAGATAT). Sanger sequencing was performed at Eurofins Genomics GmbH (Ebersberg, Germany).

### Cell viability assay

Cell viability was analyzed using the XTT cell proliferation Kit II (Roche Diagnostics, Basel, Switzerland) according to the manufacturer's instructions. Drug treatment was started 24 h after cell seeding. The absorbance was measured after 72 h of treatment and normalized to the respective untreated control cells.

### Example 1 Identification of clinically relevant bortezomib resistance mutations in Psmb5.

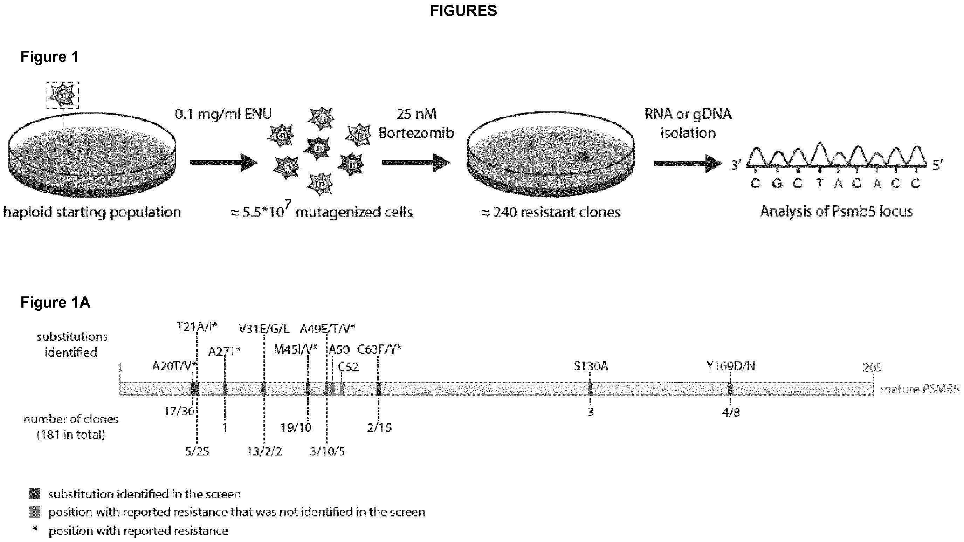

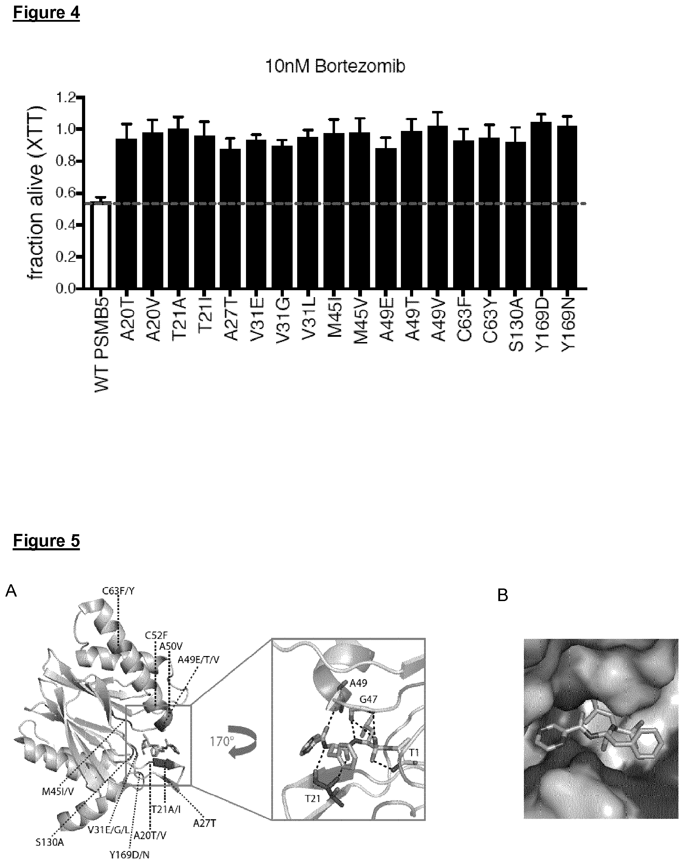

To identify variants that confer resistance to the proteasome inhibitor bortezomib, we performed an unbiased forward genetic screen in haploid mouse embryonic stem cells using chemical mutagenesis as previously described (*Elling et al*., *2011*; Figure 1). After chemical mutagenesis, AN3-12 cells resistant clones were selected with 25 nM bortezomib for three weeks. Therefore, the Psmb5 locus of 201 randomly selected resistant clones was sequenced (Figure 1).
We identified the causative mutations in 181 clones translating into 18 different amino acid substitutions at 9 positions in PSMB5 (Figure 1A and Figures 6 - 8).
A viability assay confirmed the bortezomib resistance (Figure 4). The mutant cell lines were up to 2-fold more resistant to 10 nM bortezomib compared to the wildtype control.
Most of the identified mutations were reported previously in patient-derived material or in bortezomib-resistant cell lines. Strikingly, we found three residues that were not implicated before (V31, S130, Y169).
Notably, all identified amino acid substitutions, with the exception of C63F/W/Y/, cluster in the bortezomib binding pocket of PSMB5 (Figure 5). Most of the protein-compound interaction surface was found to be mutated. Additional to T1 and G47, the identified amino acids T21 and A49 form hydrogen bonds with bortezomib. Also, the other identified amino acids are in close proximity to bortezomib. Therefore, substitution of R19, A20, T21, A22, V31, M45, A49, A50, C52, C63, S130, Y169, or G183 changes the chemical properties or the size of the binding pocket, thereby interfering with bortezomib binding. The substitution of alanine 49 with valine results in an increased size of the functional group, leading to steric hindrance of bortezomib binding (Figure 13A). The T21A substitution increases the size of the binding pocket, therefore probably destabilizing bortezomib binding (Figure 13B).

C63Y was previously described to induce conformational changes in the β5 subunit, thereby resulting in bortezomib resistance.

Taken together, the mutagenesis approach recovers known clinically relevant mutations, and also identifies novel bortezomib resistance alleles in Psmb5.

Using a similar screening approach as described above using ixazomib instead of bortezomib, two further point mutations in the PSMB5 gene were identified that results in amino acid substitution at the residues A22 and A50. As ixazomib is chemically very similar to bortezomib, these variants confer most probably resistance also to bortezomib.

Table 1 contains the details about the positions of the mutant nucleotides in the PSMB5 gene and the corresponding amino acid substitutions in the sequence of mature polypeptide β5 in mouse and human.

### Example 2 : PSMB5 mutants identified in the screen display varying degrees of resistance to other proteasome inhibitors used in the clinics.

Due to the success of bortezomib (e.g. VELCADE®) in the therapy of multiple myeloma (MM), a new generation of proteasome inhibitors has been subsequently developed. These novel proteasome inhibitors are thought to show greater activity in relapsed disease, to overcome bortezomib resistance, and to reduce toxicity (polyneuropathy). To elucidate the effect of acquired bortezomib resistance on the effectiveness of other proteasome inhibitors, we treated the isolated PSMB5 mutant cell lines with ixazomib (another boronic acid), carfilzomib (e.g. KYPROLIS®), or oprozomib (both are bulkier epoxyketones). This experimental setup mimics the situation in the clinics, where MM patients receive different proteasome inhibitors during the course of disease.

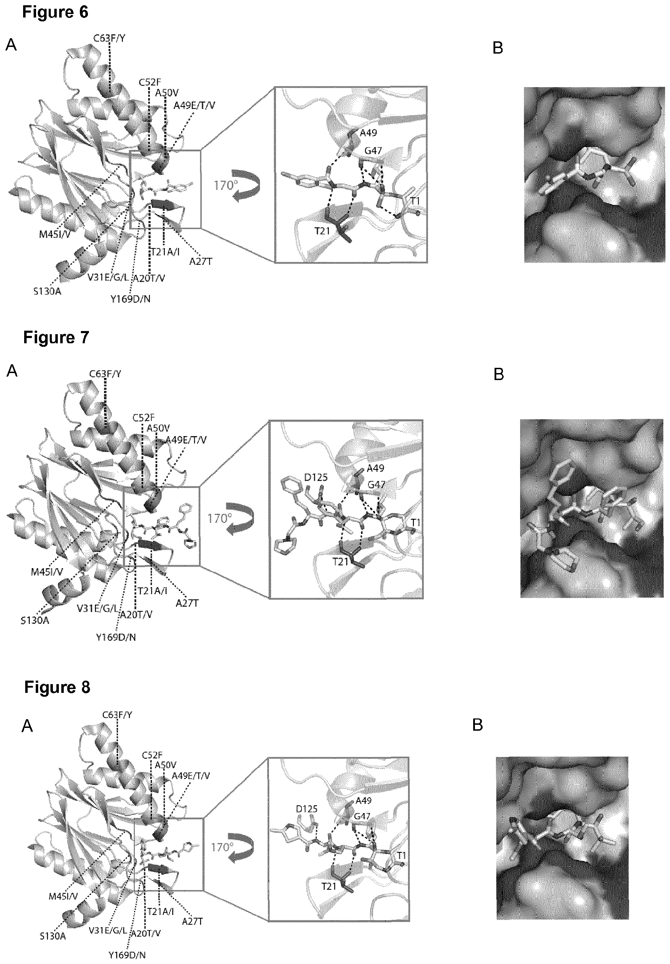

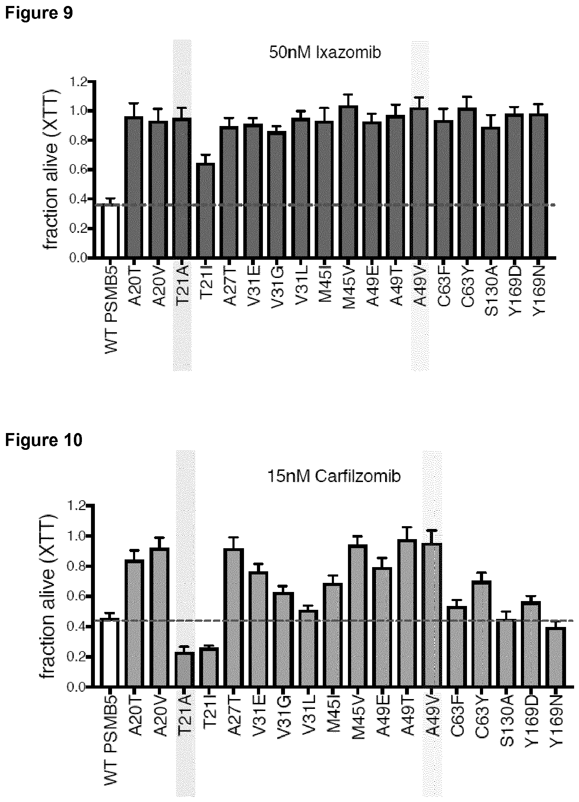

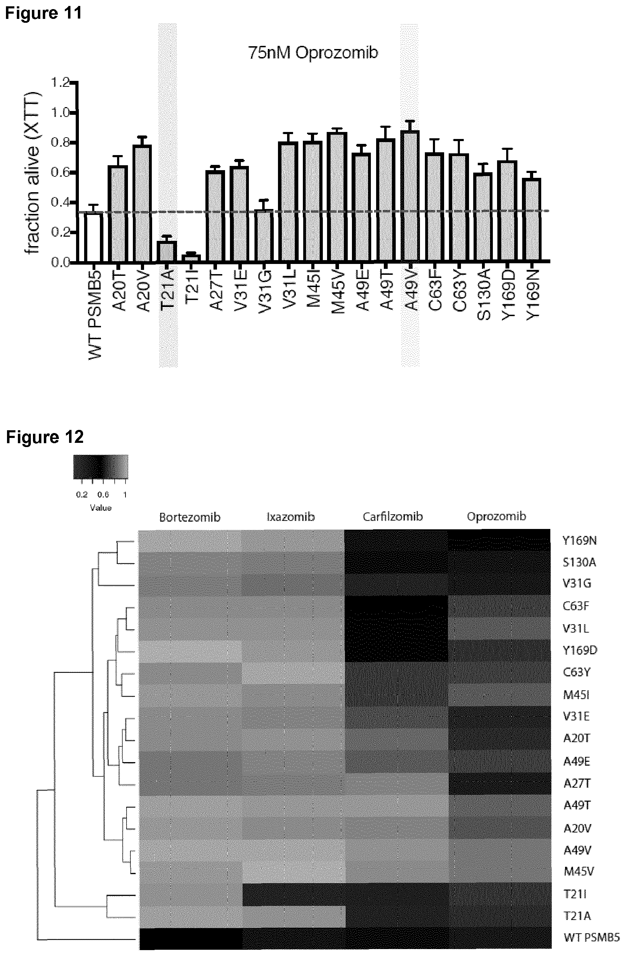

As shown in Figures 6 - 8, all of the tested proteasome inhibitors bind to the active site of PMSB5 and interfere with the catalytic N-terminal threonine residue. Consistent with the compound structures, all bortezomib resistant PSMB5 mutant cell lines were resistant to 50 nM ixazomib (Figure 9). However, treatment with 15 nM carfilzomib or 75 nM oprozomib resulted in varying degrees of resistance in the different mutants (Figure 10 - 11). While T21 substitutions resulted in carfilzomib and oprozomib-hypersensitivity (T21A is highlighted in pink), A49 substitution caused resistance to all proteasome inhibitors tested in this study (A49V is highlighted in orange). We used the results of the viability assays to cluster the mutants according to their level of resistance (Figure 12): A20, A27, M54, and A49 substitutions were resistant to all proteasome inhibitors tested, with slight variabilities regarding oprozomib. Substitutions in positions C63, S130, or Y169 showed a partially maintained response to carfilzomib, implicating carfilzomib as a potential treatment option for patients with these substitutions. Notably, the C63Y substitution was identified in a MM patient upon bortezomib treatment. Furthermore, the resistance pattern of T21 substitutions implicates carfilzomib and oprozomib as the possible second-line agents of choice. Taken together, we uncovered PSMB5 mutations that result in bortezomib resistance, but display varying responses to other proteasome inhibitors, opening a possibility for individualized treatment strategies in multiple myeloma.

**Table 1: Overview of the identified mutations of the PSMB5 gene and their consequence on amino acid level. All mutations have the same consequence on the amino acid sequence of mature polypeptide β5 in human (Seq ID No. 7) and mouse (Seq ID No. 14) except for the mutation C233A, which causes the substitution R19L in mouse.**

| **Nucleotide mutation** | | | **Amino acid substution** | |
|---|---|---|---|---|
| **Position on PSMB5 gene** (SEQ ID No 1) | **Wildtype nucleotide** | **Mutant nucleotide** | **Pro-polypeptide β5** (SEQ ID No 8) | **Mature polypeptide β5** (SEQ ID No 7) |
| 233 | C | A | R78M | R19M |
| 235 | C | T | A79T | A20T |
| 236 | G | A | A79V | A20V |
| 238 | T | C | T80A | T21A |
| 239 | G | A | T80I | T21I |
| 241 | C | T | A81T | A22T |
| 256 | C | T | A86T | A27T |
| 268 | C | A | V90L | V31L |
| 269 | A | T | V90E | V31E |
| 269 | A | C | V90G | V31G |
| 310 | T | C | M104V | M45V |
| 312 | C | A | M104I | M45I |
| 322 | C | A | A108S | A49S |
| 322 | C | T | A108T | A49T |
| 323 | G | T | A108E | A49E |
| 323 | G | A | A108V | A49V |
| 326 | G | A | A109V | A50V |
| 332 | C | A | C111F | C52F |
| 365 | C | A | C122F | C63F |
| 365 | C | T | C122Y | C63Y |
| 366 | A | C | C122W | C63W |
| 565 | A | C | S189A | S130A |
| 682 | A | C | Y228D | Y169D |
| 682 | A | G | Y228H | Y169H |
| 682 | A | T | Y228N | Y169N |
| 725 | C | T | G242D | G183D |

### Example 3: Structural changes in the active site of PSMB5 explain varying effectiveness of the different proteasome inhibitors in A49V and T21A mutants.

To better understand our findings, we modelled the identified substitutions using the mutagenesis tool of PyMOL in the structure of human polypeptideβ5, which had previously been crystallized with all proteasome inhibitors used in this study (Figure 5B, Figures 6 - 8). The A49V substitution in the β5 polypeptide resulted in resistance to all proteasome inhibitors tested (Figures 9 - 11). Here, replacement of alanine by the larger valine is expected to occupy more space. A49 forms a hydrogen bond with the bound proteasome inhibitors. In our model this bond remains intact as it is formed with the protein backbone (Figure 13A, black dashed lines). Thus, the resistance to the proteasome inhibitors was most likely not caused by a loss of the hydrogen bond. Instead, the replacement of A49 with valine caused steric clashes indicated by red disks with all proteasome inhibitors and additionally with S129 of the β6 subunit of the proteasome (Figure 13A). These steric changes might interfere with proteasome inhibitor binding.

In contrast to A49V, the T21A substitution conferred resistance only to the boronic acids bortezomib and ixazomib. T21A mutants were even hypersensitive to carfilzomib and oprozomib (Figures 9 - 11). Threonine is an uncharged, but polar amino acid with a bigger side chain than alanine. Like A49, the T21 backbone forms hydrogen bonds with the proteasome inhibitors. Therefore, also these hydrogen bonds can still be formed after the substitution (Figure 13B, black dashed lines). Since the T21A substitution enlarges the binding pocket, we speculate that the affinity of bortezomib and ixazomib to the active site was reduced by the substitution (Figure 13B). The epoxyketones carfilzomib and oprozomib are bulkier proteasome inhibitors that bind the β5 subunit irreversibly. Therefore, it is likely that the T21A substitution was hypersensitive to carfilzomib and oprozomib because these proteasome inhibitors can access the enlarged binding pocket more efficiently. Since they bind irreversibly, the binding cannot be destabilized by a larger binding pocket once it is in place. Taken together, this structural analysis supports the differential response of the investigated substitutions to second-generation proteasome inhibitors.

### Example 4: Treatment decision for patients on the basis of Bortezomib resistance.

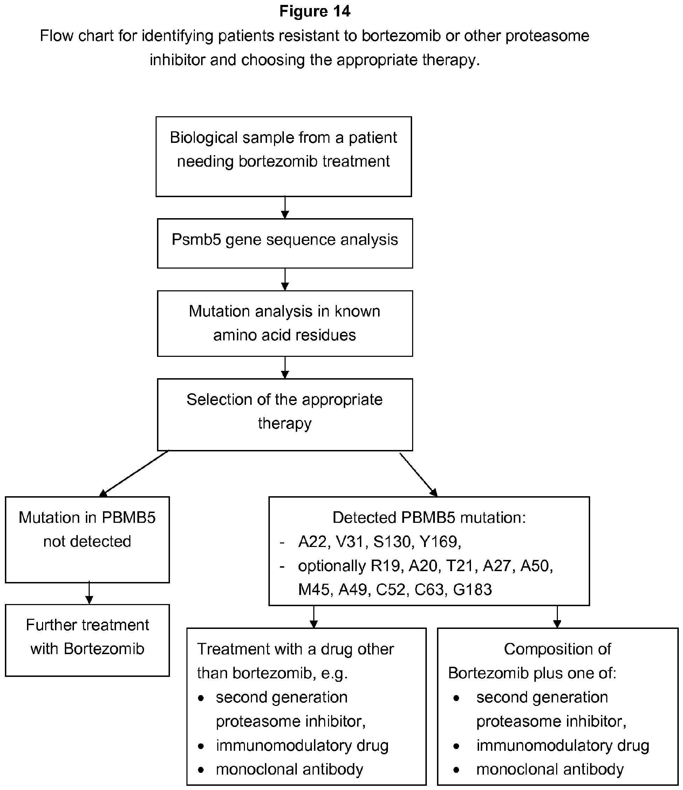

A patient is identified as having a disease that is curable with bortezomib, and thus proteasome inhibitor sensitive. Then, the patient is treated with bortezomib according to clinical practice. During and /or after the treatment with bortezomib, a biological sample containing diseased cells (i.e. bone marrow cells) is collected from the patient and subjected to the analysis of Psmb5 sequence, for instance as described in the method section (page 49.; Figure 14). Results are then interpreted in order to reveal the presence of a mutation in the PSMB5 gene that results in a substitution in the amino acid sequence of mature β5 in at least one of the amino acid residues A22, V31, S130, Y169, and optionally in the amino acids residues R19, A20, T21, A27, M45, A49, C52, C63, and G183. The patient is further treated with bortezomib, or another drug against which the detected substitution is known to not cause resistance, or a composition of bortezomib and a further drug, for example a second generation proteasome inhibitor, or an immunomodulatory drug, or a monoclonal antibody. The treatment with bortezomib or a composition of bortezomib and Ixazomib or Carfilzomib or Oprozomib can be selected with the help of the heatmap of Figure 12.

## Claims

1. An *in vitro* method for identifying resistance to bortezomib treatment in a patient prior, during or after bortezomib treatment, comprising:
a) obtaining a biological sample containing diseased cells from said patient, and
b) detecting a substitution in the amino acid sequence of the polypeptide β5 (PSMB5) in at least one of the amino acid residues A22, V31, S130 and Y169, wherein said substitution inhibits the binding of bortezomib to the active site of the polypeptide β5, thereby identifying said patient as being resistant to bortezomib treatment.

2. The method according to claim 1, wherein the step b) further comprises detecting a substitution in the amino acid sequence of the polypeptide β5 in at least one of the amino acid residues R19, A20, T21, A27, M45, A49, A50, C52, C63, and G183.

3. The method according to claim 1 further comprising after step a) the steps a') and a"):
a') determining the nucleic acid sequence of the PSMB5 gene encoding the polypeptide β5, and
a") detecting a mutation in the nucleic acid sequence of the PSMB5 gene that results in a substitution in the amino acid sequence of the polypeptide β5 in at least one of the amino acid residues A22, V31, S130 and Y169.

4. The method according to claim 2 or 3 further comprising after step a) the steps a') and a"):
a') determining the nucleic acid sequence of the PSMB5 gene encoding the polypeptide β5, and
a") detecting a mutation in the nucleic acid sequence of the PSMB5 gene that results in a substitution in the amino acid sequence of the polypeptide β5 in at least one of the amino acid residues R19, A20, T21, A27, M45, A49, A50, C52, C63, and G183.

5. The method according to any one of the claims 1 - 4, wherein the patient is suffering from a disease selected from the group comprising or consisting of multiple myeloma, mantle cell lymphoma, non-Hodgkin's lymphoma TEMPI syndrome, light chain deposition disease (LCDD), IgG₄-related disease, and scleromyxedema.

6. The method according to any one of the claims 1 - 5, wherein the biological sample containing diseased cells of step a) is obtained from bone marrow, whole blood, mononuclear cells, or plasma cells from said patient.

7. The method according to claim 3 or 4, wherein determining the nucleic acid sequence comprises DNA sequencing.

8. A composition of bortezomib and at least one further drug suitable for the treatment of a patient suffering from a disease selected from the group comprising multiple myeloma, mantle cell lymphoma, non-Hodgkin's lymphoma, TEMPI syndrome, light chain deposition disease (LCDD), IgG₄-related disease, and scleromyxedema, wherein a substitution in the amino acid sequence of the polypeptide β5 in at least one of the amino acid residues A22, V31, S130 and Y169 does not result in resistance to that further drug.

9. The composition according to claim 8, wherein a substitution in the amino acid sequence of the polypeptide β5 in at least one of the amino acid residues R19, A20, T21, A27, M45, A49, A50, C52, C63, and G183 of the polypeptide β5 does not result in resistance to that further drug.

10. The composition according to claim 8 or 9, wherein the further drug is a second generation proteasome inhibitor selected from the group consisting of carfilzomib (PR-171), oprozomib (ONX-0912), ixazomib (MLN9708 / MLN2238), delanzomib (CEP-18770), marizomib (NPI-0052), Syringolins, and Syringolin analogues.

11. The composition according to claim 8, 9 or 10, wherein the further drug is an immunomodulatory drug selected from the group consisting of lenalidomide, pomalidomide or thalidomide, or wherein the further drug is a monoclonal antibody selected from the group consisting of anti-IL6, anti-CXCR4, anti-CD38 or anti SLAMF7 Elotozumab, or a histone deacetylase inhibitor.

12. A diagnostic kit for identifying resistance to bortezomib treatment in a patient suffering from a proteasome inhibitor sensitive disease comprising:
i) an extraction system comprising materials to isolate RNA or gDNA,
ii) sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues A22, V31, S130 and Y169,
iii) means for revealing the polymerase chain reaction amplification of step (ii).

13. The diagnostic kit according to claim 12, further comprising:
iv) further sensitive oligonucleotide primers to perform a quantitative polymerase chain reaction of the isolated RNA or gDNA to amplify a nucleic acid sequence containing a mutation of the PSMB5 gene that results in a substitution in the amino acid sequence of the polypeptide β5 in at least one of the amino acid residues R19, A20, T21, A27, M45, A49, A50, C52, C63, and G183.

14. Use of the diagnostic kit of claim 12 or 13 for identifying resistance to bortezomib treatment in a patient having a substitution in the amino acid sequence of the polypeptide β5 in at least one of the amino acid residues A22, V31, S130 and Y169.

15. Use of the diagnostic kit of claim 12 or 13 for identifying resistance to bortezomib treatment in a patient, having a substitution in the amino acid sequence of the polypeptide β5 in at least one of the amino acid residues R19, A20, T21, A27, M45, A49, A50, C52, C63, and G183.
